# EUROPÄISCHE PATENTSCHRIFT

(11) **EP 1 373 270 B1**
(45) Veröffentlichungstag und Bekanntmachung des Hinweises auf die Patenterteilung: **06.06.2007**
(21) Anmeldenummer: 02716759.2
(22) Anmeldetag: 11.02.2002
(51) Int. Cl.: C07D 487/04, A61P 17/06, A61Q 17/04, A61K 8/49

(54) **TRIZYKLISCHE CHINOXALINDERIVATE ALS UV-FILTER**
TRICYCLIC QUINOXALINE DERIVATIVES AS A UV FILTER
DERIVES DE QUINOXALINE TRICYCLIQUE UTILISES COMME FILTRE UV

(30) Priorität: 09.03.2001 DE 10111728
(43) Veröffentlichungstag der Anmeldung: 02.01.2004
(73) Patentinhaber: Merck Patent GmbH, 64293 Darmstadt (DE)
(72) Erfinder: PFLÜCKER, Frank, 64297 Darmstadt (DE); SCHWARZ, Michael, 64331 Weiterstadt (DE); SCHOLZ, Volker, 64295 Darmstadt (DE); NEUNHOEFFER, Hans, 64367 Mühltal (DE)
(86) Internationale Anmeldenummer: PCT/EP2002/001402
(87) Internationale Veröffentlichungsnummer: WO 2002/072583

(56) Entgegenhaltungen:
- WO-A-01/68047
- WO-A-89/02739
- DE-A- 4 302 796
- US-A- 3 935 312
- SCHOLZ V ET AL: "A STRATEGY FOR THE DEVELOPMENT OF UV-FILTERS AND CONTROL OF THEIR ABSORPTION PROPERTIES" SOFW-JOURNAL SEIFEN, OELE, FETTE, WACHSE, VERLAG FUR CHEMISCHE INDUSTRIE, H. ZIOLKOWSKY K.G. AUGSBURG, DE, Bd. 127, Nr. 4, 1. April 2001 (2001-04-01), Seiten 3-4,6,8-11, XP001005065 ISSN: 0942-7694
- DATABASE CROSSFIRE BEILSTEIN [Online] Beilstein Institut zur Förderung der Chemischen Wissenschaften, Frankfurt am Main, DE; Database accession no. 29054,36252,234949,259836,273681,299802,29 9962 XP002201320 & JOURNAL OF THE INDIAN CHEMICAL SOCIETY, Bd. 9, 1932, Seiten 527-530,
- GALLOS, J. ET AL.: "Synthesis of imidazo[4,5-b]quinoxaline-3-oxides from reactions of furazano3,4-bquinoxaline 1-oxides with nitrones and diazo compounds" JOURNAL OF HETEROCYCLIC CHEMISTRY, Bd. 30, Nr. 4, 1993, Seiten 917-912, XP001079476 ISSN: 0022-152x
- BUU-HOI, N.P. ET AL: "Dérivés deu flavazole (+H-pyrazolo[3,4-b]quinoxaline). I. Chimie, Physico-chimie et pharmacologie du flavazole et de quelques-uns de ses dérivés substitués" CHIMIE TH¹RAPEUTIQUE, Bd. 6, Nr. 2, 1971, Seiten 245-250, XP001079331

## Beschreibung

Die Erfindung betrifft die Verwendung von Chinoxalinderivaten als photostabile UV-Filter in kosmetischen und pharmazeutischen Zubereitungen zum Schutz der menschlichen Epidermis oder menschlicher. Haare gegen UV-Strahlung, vor allem im Bereich von 280-400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Zubereitungen soll eine Schädigung der Keratinfaser durch UV-Strahlen vermindert werden.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, dass die Haut ihre Elastizität verliert und sich Falten bilden und führen somit zu einer frühzeitigen Alterung. Manchmal kann man auch Dermatosen beobachten, und im extremen Fall kommt es zum Auftreten von Hautkrebs.

Aufgrund dieses Wissens hat sich auch im Sonnenschutz einiges geändert. Während noch vor einigen Jahren das Hauptziel in einem erythemhemmenden UV-B-Schutz bestand, wird inzwischen ein Schutz gegen UV-A-Strahlung in Sonnenschutz Zubereitungen mit eingeschlossen.

Die UV-A-Strahlung ist im Wesentlichen Auslöser für die Pigmentierung der Haut.

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenlänge von weniger als 400 nm gebildet. Bekannt ist auch, dass durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt. All diese Erkenntnisse lassen daher die Entwicklung effizienter Filtersubstanzen für den UV-A- und auch für den UV-B-Bereich notwendig erscheinen.

Es besteht ein wachsender Bedarf an Lichtschutzmitteln für kosmetische und pharmazeutische Zubereitungen, die vor allem als UV-A- Filter dienen können und deren Absorptionsmaxima deshalb im Bereich von ca. 320 bis 400 nm liegen sollten. Ferner besteht auch ein Bedarf an einem Breitbandschutz, also UV-A- und UV-B-Schutz, im Bereich von 280-400 nm.

Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen oder auch in Wasser, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber oft mit den bisher verfügbaren UV-A und UV-B absorbierenden Lichtschutzmitteln nur unzureichend gewährleistet.

Es gibt im Stand der Technik zwar verschiedene Ansätze, die Photostabilität von guten Lichtschutzfiltern, wie z.B. von Dibenzoylmethanen, durch Kombination mit verschiedenen UV-B-Filtern zu verbessern (FR 2 440 933), oder auch durch Zugabe bestimmter Substanzen die UV-Filter zu stabilisieren (EP 0514491), jedoch sind damit noch keine ausreichenden Lösungen vorhanden.

Weiterhin werden in den Offenlegungsschriften DE 197 46 656 und EP 0 852 137 Substanzklassen wie 4,4-Diarylbutadiene bzw. Verbindungen mit einer R⁴-NH-CR³=CR¹R²-Struktur als neue Lichtschutzfilter vorgeschlagen, welche jedoch der Nachfrage nach geeigneten Verbindungen für den UV-A- und UV-B-Bereich nicht ausreichend genügen.

Es bestand daher die Aufgabe, eine neue Strukturklasse als Lichtschutzmittel für kosmetische und pharmazeutische Zwecke zu finden, die im UV-A- und/oder UV-B-Bereich absorbieren, die photostabil sind, eine geringe Eigenfarbe, d.h. eine scharfe Bandenstruktur aufweisen, eine hohe Extinktion haben und je nach Substituent in Öl oder Wasser löslich sind.

In der älteren Anmeldung WO 01/68047 wurde gefunden, dass Chinoxalinderivate mit verschiedensten Resten hervorragende UV-B- und/oder UV-A- Eigenschaften besitzen und die vorstehend beschriebenen Anforderungen in hohem Maße erfüllen.

Aus der online Datenbank Crossfire Beilstein sind die Chinoxalin-derivate 2-(-4-methoxy-phenyl)-1H-imidazo[4,5-b]chinoxalin, 4-(-1H-imidazo[4,5-b]chinoxalin-2-yl)-resorcin, 2-p-toluyl-1H-imidazo[4,5-b]chinoxalin, 2-(-1H-imidazo[4,5-b]chinoxalin-2-yl)-phenol, 4-(1H-imidazo[4,5-b]chinoxatin-2-yl)-N,N-dimethylanilin, 2-(4-nitro-phenyl)-1H-imidazo[4,5-b]chinoxalin und 2-(3-nitrophenyl)-1H-imidazo[4,5-b]chinoxalin bekannt.

Aus US 3,935,312 sind verschiedene 2-(Halo)alkyl-1H-imidazo[4,5-b]chinoxalin-Derivate, die sich zur Bekämpfung von Pilzen eignen, sowie die entsprechende Verwendung bekannt.

Aus J.K. Gallos, E. Malamidou-Xenikaki, J. Heterocyclic Chem. 30 (193) 917-920 ist die Synthese von lmidazo[4,5-b]chinoxalin-3-oxiden bekannt. Dabei wird auch die Synthese von 1-Methoxy-2-phenylimidazo[4,5-b]chinoxalin und 2-Ethoxycarbonyl-6,7-dimethylimidazo[4,5-b]chinoxalin offenbart.

Aus WO 89/02739 sind 4-Aminopyridin-Derivate bekannt, die sich zur Behandlung der Alzheimer Krankheit eignen.

N.P. Buu-Hoi, J.-N. Vallat, G. saint-Ruf und G. Lambelin, Chimie Therapeutique Bd. 6, No.2 (1971) 245-250 beschreiben verschiedene 1H-Pyrazolo[3,4-b]chinoxaline und deren pharmakologische Eigenschaften.

Jetzt wurde gefunden, dass spezielle Derivate nach Formel 1 zur Lösung dieser Aufgabe in besonderem Maße geeignet sind.

Ein erster Gegenstand der vorliegenden Erfindung ist daher die Verwendung von Verbindungen ausgewählt aus einer der Formeln IV, V, VI oder VII, 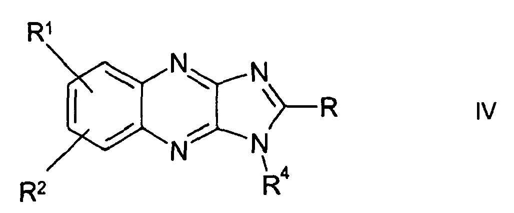 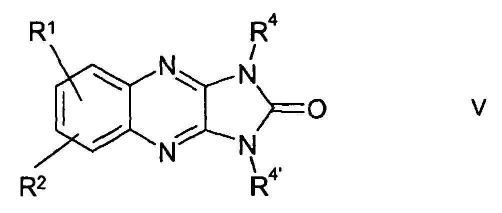 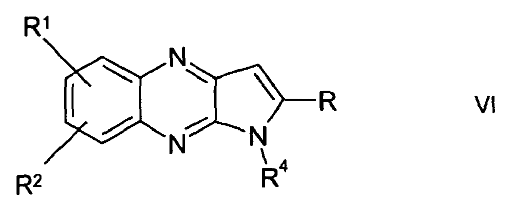 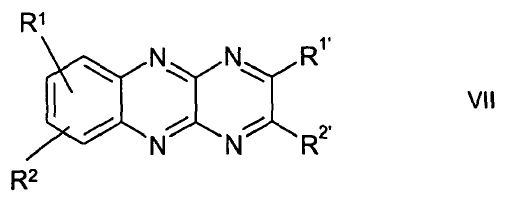 wobei
- R, R¹, R², R^{1'} und R^{2'}: jeweils unabhängig voneinander H, Alkyl, Alkoxy, Alkenyl oder Alkinyl jeweils mit bis zu 20 C-Atomen, Cycloalkyl, Cycloalkoxy, Cycloalkenyl oder Bicyclische Systeme jeweils mit bis zu 10 C-Atomen, wobei in allen diesen Gruppen auch ein oder mehrere Wasserstoffatome durch Sub¹ substituiert und/oder eine oder zwei CH₂-Gruppen durch C=O ersetzt sein können und in den cyclischen Systemen 1 bis 3 Heteroatome wie S, N und/oder O enthalten sein können, Hal, OH, NO₂, -(CR⁵R⁶)ₙ-NR⁵R⁶, -SO-R⁵, -NR⁵-SO-R⁶, -(CR⁵R⁶)ₙ-N=CR⁵R⁶, -(CR⁵R⁶)ₙ-CR⁵=NR⁵, -SO₂-R⁵, -SR⁵, -(CR⁵R⁶)ₙ-NHCOR⁵, -(CR⁵R⁶)ₙ-NHCOOR⁵,
wasserlöslich machende Substituenten, ausgewählt aus der Gruppe bestehend aus Carboxylat-, Sulfonat- oder Ammoniumresten, COR⁵, COOR⁵, CON⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Si(Alkyl)₃, -Si(Alkyl)₂H, -Het, -NHHet, -OHet oder -(CR⁵R⁶)ₙ-Het,
R¹ und R² bzw. R^{1'} und R^{2'} jeweils zusammen auch mit Kohlenstoffatomen, an die sie gebunden sind, gemeinsam einen ungesättigten, teilweise oder vollständig gesättigten 4-, 5-, 6- oder 7-Ring bilden können, der gegebenenfalls Heteroatome wie S, N und/oder O enthalten kann, weiter anelliert und/oder auch ein- oder mehrfach substituiert sein kann,
- Sub¹: Hal, Hydroxy, Cyano, Amino, Nitro, C₁-C₄ -Alkylamino, C₁-C₄-Dialkylamino, C₁-C₄-Alkyl oder C₁-C₄ -Alkoxy, COOH oder COOAlkyl,
- Hal: Fluor, Chlor, Brom, Jod,
- n: 0, 1, 2, 3 oder 4,
- R⁵, R⁶: jeweils unabhängig voneinander H, Alkyl, Alkenyl, Alkinyl, jeweils mit bis zu 20 C-Atomen, Cycloalkyl, Cycloalkenyl, Bicyclische Systeme, jeweils mit bis zu 10 C-Atomen, wobei diese Reste bis dreifach durch Sub¹ substituiert vorliegen und/oder ein oder zwei CH₂-Gruppen durch C=O ersetzt sein können, und die cyclischen Systeme auch 1 bis 3 Heteroatome wie S, N, und/oder O enthalten können,

-(CR'R")ₙ-Ar oder -(CR'R")ₙ-Het,

die Reste R⁵ und R⁶ auch untereinander, jeweils zusammen mit C-Atomen, an die sie gebunden sind, gemeinsam einen ungesättigten, teilweise oder vollständig gesättigten 4-, 5-, 6- oder 7-Ring bilden können, der gegebenenfalls Heteroatome wie S, N und/oder O enthalten kann, auch ein- oder mehrfach substituiert und/oder weiter anelliert sein kann,
- R' und R": jeweils unabhängig voneinander H oder C₁-C₄-Alkyl, wobei auch ein oder zwei CH₂-Gruppen durch C=O ersetzt sein können,
- Ar: ein unsubstituierter oder ein- oder mehrfach substituierter aromatischer Ring oder kondensierte Ringsysteme mit 6 bis 18 C-Atomen, worin auch ein oder zwei CH-Gruppen durch C=O ersetzt sein können,
- Het: ein unsubstituierter oder ein-oder mehrfach substituierter heteroaromatischer Ring mit 5 bis 7 Ringgliedern oder ein kondensiertes Ringsystem, wobei als Heteroatome ein oder mehrere N-, S- und/oder O-Atome enthalten sind und worin auch ein oder zwei CH-Gruppen in α- oder β-Position zu den Heteroatomen durch C=O ersetzt sein können,
- R⁴ und R^{4'}: jeweils unabhängig voneinander stehen für H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome wie S, N, und/oder O enthalten können,
- Sub²: Hal, Hydroxy, Cyano, Amino, Nitro, C₁-C₄-Alkyl oder C₁-C₄ -Alkoxy, COR⁵, COOR⁵, OAr, OHet, -(CR⁵R⁶)ₙ-Ar oder -(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁶)=O oder R⁵OP(-OR⁶)=O, bedeuten,
als photostabile UV-Filter.

Die erfindungsgemäßen Verbindungen der Formel IV-VII werden im folgenden auch als Chinoxalinderivate oder Verbindungen gemäß Formel I bezeichnet, obwohl damit auch Verbindungen umfaßt sind, die im strengen chemischen Sinne keine Chinoxalinderivate darstellen.

Die Herstellung der Verbindungen gemäß Formel I kann weitgehend Literaturanalog erfolgen. Herstellfahren sind beispielsweise in D. Ames, M. Brohi; J. Chem. Soc perkin. Trans. I (1980) 1384-1389 beschrieben. Zur detailierten Offenbarung der Herstellung erfindungsgemäßer Verbindungen nach Formel I wird insbesondere auch auf den Bespielteil dieser Anmeldung (Beispiele A - G) verwiesen.

Die Verbindungen nach Formel 1 zeigen hervorragende UV-absorbierende Eigenschaften sowohl im UV-A-Bereich als auch bei Vorhandensein einer zusätzlichen chromophoren Gruppe im UV-B-Bereich, wodurch ein Breitbandschutz gewährt wird. Ebenso kann leicht durch die Wahl geeigneter Substituenten die Löslichkeit der Substanzen in Wasser oder in kosmetischen Ölen induziert werden. Lipophile, d.h. die Öllöslichkeit der Verbindungen der Formel I verstärkende Rest sind beispielsweise aliphatische oder cycloaliphatische Reste, insbesondere Alkylreste mit bis zu 20 C-Atomen, Alkoxy-, Mono- und Dialkylamino-, Alkoxycarbonyl, Mono- und Dialkylaminocarbonyl, Mono- und Dialkylaminosulfonylreste, ferner auch Cyan-, Nitro-, Brom-, Chlor-, Iod- oder Fluorsubstituenten.

Hydrophile, d.h. die Wasserlöslichkeit der Verbindungen der Formel I ermöglichende Reste sind z.B. Carboxy- und Sulfoxyreste und insbesondere deren Salze mit beliebigen physiologisch verträglichen Kationen, wie die Alkalisalze oder die Trikalkylammioniumsalze.

Die Alkylreste in den Resten R und R¹ bis R⁶ besitzen bis zu 20 C-Atomen und können unverzweigt oder verzweigt vorliegen und bedeuten demnach bevorzugt Methyl, Ethyl, n-Propyl, i-Propyl, Butyl, sek.-Butyl, i-Butyl, tert.-Butyl, Pentyl, 1-Methylbutyl, 2-Methylbutyl, 3-Methylbutyl, Hexyl, 1-Methylpentyl, 2-Methylpentyl, Heptyl, Octyl, Nonyl, Decyl, Undecyl oder Dodecyl, ferner bedeuten sie auch 2,2-Dimethylpropyl, 1-Ethylpropyl, 3-Methylpentyl, 4-Methylpentyl, 1,1-Dimethylpropyl, 1,2-Dimethylpropyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1-Ethylhexyl, 2-Ethylhexyl, Tridecyl, Tetradecyl, Pentadecyl, Hexadecyl, Heptadecyl, Octadecyl, Nonadecyl oder Eicosyl.

Als bevorzugte Alkenylreste seien verzweigte und unverzweigte Alkenylketten mit vorzugsweise bis zu 10 C-Atomen genannt: Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, Octenyl, Nonenyl oder Decenyl.

Als Alkinylreste kommen vorzugsweise verzweigte oder unverzweigte Alkinylketten mit bis zu 10 C-Atomen in Betracht, wie z.B. Ethinyl, Propinyl, Butinyl, i-Butinyl, Pentinyl, Hexinyl, Heptinyl oder Octinyl.

Als Cycloalkylreste seien bevorzugt verzweigte oder unverzweigte C₃-C₁₀-Cycloalkylketten wie Cyclopropyl, Cyclobutyl, Cyclopentyl, Cyclohexyl, Cycloheptyl, Cyclooctyl, 1-Methylcyclopropyl, 1,2-Dimethylcyclopentyl, 1-Methyl-2-ethylcyclopropyl, Cyclononyl oder auch Cyclodecyl genannt.

Als Alkoxyreste kommen verzweigte oder unverzweigte Alkoxyketten mit bis zu 20 C-Atomen, vorzugsweise mit bis zu 12 C-Atomen, insbesondere bevorzugt mit 1 bis 8 C-Atomen in Frage, wie z.B. Methoxy, Ethoxy, Propoxy, i-Propoxy, Butoxy, 1-Methylpropoxy, 2-Methylpropoxy, Pentoxy, 1,1-Dimethylpropoxy, 1-Methylbutoxy, 3-Methylbutoxy, 2-Methylbutoxy, Hexoxy, Heptoxy, oder auch Octoxy.

Als Cycloalkylreste seien für R und R¹ bis R⁶ bevorzugt verzweigte oder unverzweigte Cycloalkylketten mit 3 - 10 C-Atomen wie Cyclopropyl, Cyclobutyl, Cyclopentyl, Cyclohexyl, Cycloheptyl, 1-Methytcyclopropyl, 1-Ethylcyclopropyl, 1-Propylcyclopropyl, 1,2-Dimethylcyclopropyl, 1-Methylcyclohexyl, 1,3-Dimethylcyclohexyl, Cyclooctyl, Cyclononyl oder Cyclodecyl genannt.

Als Cycloalkenylreste seien bevorzugt verzweigte oder unverzweigte C₃-C₁₀-Cycloalkenylketten mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, Cyclononenyl oder Cyclodecenyl genannt.

Als Bicycloalkyl- oder Bicycloalkenylreste seien gesättigte oder ungesättigte bicyclische Ringsysteme mit vorzugsweise bis zu 10 C-Atomen, vorzugsweise bicyclische Terpene wie Pinan-, Pinen-, Bornan-, Campherderivate, Decalin oder auch Adamantan genannt.

In diesen cyclischen Systemen können auch 1 bis 3 Heteroatome wie Schwefel, Stickstoff oder Sauerstoff enthalten sein. Beispielhaft seien hierzu Ringsysteme wie Piperidin-, Pyrrolidin-, Pyrazin- oder Pyridazin-, Pyrimidin-, Morpholin-, Tetrahydro-furan-, Dihydrofuran-, Thiolan-, Piperazin-, Thiazolidin- oder auch Oxazolidingruppen genannt.

In den vor- und nachstehenden Resten, die durch Sub¹ substituiert sein können, bedeutet Sub¹ vorzugsweise Halogen, wie Fluor, Chlor, Brom oder Iod, vorzugsweise Fluor oder Chlor, weiterhin bevorzugt C₁-C₄-Alkylamino oder C₁-C₄-Dialkyamino, C₁-C₄-Alkyl oder C₁-C₄-Alkoxy oder auch Hydroxy oder Amino.

In den vor- und nachstehenden Resten, die durch Sub² substituiert sein können, bedeutet Sub² vorzugsweise Halogen, wie Fluor, Chlor, Brom oder Iod, vorzugsweise Fluor oder Chlor, weiterhin bevorzugt C₁-C₄-Alkylamino oder C₁-C₄-Dialkyamino, C₁-C₄-Alkyl oder C₁-C₄-Alkoxy oder auch Hydroxy oder Amino, ferner bevorzugt ist auch die Bedeutung COR⁵, -(CR⁵R⁶)ₙ-Ar, -(CR⁵R⁶)ₙ-Het, OAr, OHet, COOR⁵ oder auch R⁵OP(-OR⁶)=O.

Als Mono- oder Dialkylaminoreste kommen vorzugsweise Methylamino, Dimethylamino, Ethylamino, Methyl-ethylamino, Diethylamino, Propylamino, Methyl-propylamino, Dipropylamino, Ethyl-propylamino, Butylamino, Dibutylamino, Methyl-butylamino, Isopropylamino in Betracht, ferner auch 1,1-Dimethylpropylamino, Pentylamino, Hexylamino, 1-Methyl-1-ethylpropylamino, Heptylamino oder Octylamino.

In den vor- und nachstehenden Verbindungen und Formeln bedeutet Ar einen unsubstituierten oder ein- oder mehrfach substituierten aromatischen Ring oder ein kondensiertes Ringsystem mit 6 bis 18 C-Atomen, vorzugsweise mit 6 bis 10 C-Atomen, worin auch ein oder zwei CH-Gruppen durch C=O ersetzt sein können. Als insbesondere bevorzugte Gruppen seien unsubstituiertes oder substituiertes Phenyl oder Naphthyl genannt.

Het bedeutet in den vor- und nachstehenden Definitionen einen unsubstituierten oder ein- oder mehrfach substituierten heteroaromatischen Ring mit 5 bis 7 Ringgliedern oder ein kondensiertes Ringsystem mit vorzugsweise bis zu 14 Ringatomen, wobei als Heteroatome ein oder mehrere N-, S- und/oder O-Atome enthalten sind und worin auch ein oder zwei CH-Gruppen durch C=O ersetzt sein können. Soweit nicht anders definiert, haben die heterocyclischen Ringe vorzugsweise 1-13 C-Atome und 1-6 Heteratome, insbesondere 3-9 C-Atome und 1-4 Heteroatome. Beispielsweise kommen heteroaromatische Reste wie 2- oder 3-Thienyl, 2- oder 3-Furyl, 2-, 3-, oder 4-Pyridyl, Pyrimidyl, Pyrazolyl, Pyrazolonyl, Imidazolyl, Triazinyl, Pyrazinyl, Thiazolyl, Indolyl, Chinolyl, Chinoxalinyl oder Isochinolyl in Frage.

Die vorstehend beschriebenen Ar- und Het-Gruppen sind vorzugsweise unsubstituiert oder ein-, zwei- oder dreifach substituiert, wobei als Substituenten prinzipiell alle Substituenten möglich sind, solange sie keinen toxischen Einfluss auf die Gesamtverbindungen haben. Vorzugsweise kommen die als Sub² definierten Substituenten in Frage und demnach vorzugsweise folgende Substituenten: Halogen, insbesondere F oder CI, Hydroxy, Amino, Cyano, C₁-C₄-Alkyl oder C₁-C₄ -Alkoxy, COR⁵, COOR⁵, OAr, OHet, -(CR⁵R⁶)ₙ-Ar oder-(CR⁵R⁶)ₙ-Het, -(CR⁵R⁶)ₙ-NR⁵R⁶, CONR⁵R⁶, CN, O=S(-R⁵)=O, O=S(-OR⁵)=O, O=S(-NR⁵R⁶)=O oder R⁵OP(-OR⁶)=O.

Ferner ist es auch möglich, dass im Ringsystem eine CH-Gruppe durch C=N-ersetzt ist, d.h. der Substituent hat dann die Bedeutung von =N-R* , worin R* gleich R⁵ oder auch -NH-Ar ist.

Ferner sind folgende Substituenten ganz besonders bevorzugt: Fluor, Chlor,-COOH, Alkoxy mit bis zu 8 C-Atomen, -COOAlkyl mit bis zu 8 C-Atomen, -CO-Phenyl, -CO-Aryl, -CO-Het, -chinoxalinyl, -CO-NH-R⁵. Diese Aufzählung hat rein beispielhaften Charakter und soll keineswegs limitierend sein.

In den vorstehenden Definitionen können die Gruppen Ar, Het, R⁵ und/oder R⁶ ebenfalls wieder Substituenten, wie sie vor- oder nachstehend für diese Gruppen beschrieben sind, tragen.

Bevorzugt findet ein Chinoxalinderivat Verwendung das ausgewählt ist aus den Verbindungen gemäß Formel VIII, 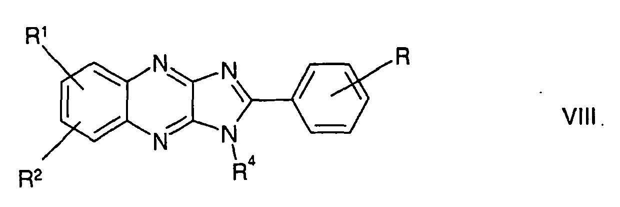 wobei,
R, R1 und R2 die oben gegebene Bedeutung haben,
R4 steht für H oder einen verzweigten oder unverzweigten C1-20-Alkylrest, in dem gegebenenfalls ein oder mehrere H-Atome durch Sub ersetzt sein können, Sub steht für H, Methyl, Ethyl oder Ethylhexyl und
R4 steht vorzugsweise für H, Methyl, Ethyl oder Ethylhexyl und
R vorzugsweise steht für einen verzweigten oder unverzweigten C1-20-Alkylrest, in dem gegebenenfalls ein oder mehrere H-Atome durch Sub ersetzt sein können. Insbesondere sind solche Verbindungen bevorzugt, bei denen R steht für Methyl, Ethyl, iso-Propyl oder tertiär Butyl. Weiterhin bevorzugt ist 1-(2-Ethyl-hexyl)-2-phenyl-imidazo[3,4-b]chinoxalin.

Insbesondere bevorzugt ist es, wenn der Phenylring nach einem der in Tabelle 1 angebenen Muster substitutiert ist.

**Tabelle 1**

| R | m | Position | 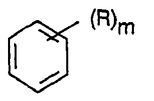 |
|---|---|---|---|
| CH₃ | 1- 3 | o/ m/ p ; m/ m/ p; o/p; m/ m | |
| C₂H₅ | 1-3 | " | |
| C₃H₇ | 1-3 | " | |
| ⁿC₄H₉ | 1-3 | " | |
| ^{t}C₄H₉ | 1-3 | " | |
| ⁱC₄H₉ | 1-3 | " | |
| C₅H₁₁ | 1-3 | " | |
| C₁₈H₃₇ | 1-3 | " | |
| (2'-Etyl)-Hexyl- | 1-3 | " | |
| OCH₃ | 1-3 | o/ m/ p ; m/ m/ p; o/p; m/ m | |
| OC₂H₅ | 1-3 | " | |
| 0C₃H₇ | 1-3 | " | |
| OC₄H₉ | 1-3 | " | |
| OC₅H₁₁ | 1 _3 | " | |
| OC₁₈H₃₇ | 1 -3 | " | |
| OCOCH₃ | 1-2 | o; m; p; o/p | |
| OCOC₂H₅ | 1-2 | " | |
| OCOC₃H₇ | 1-2 | " | |
| OCOC₄H₉ | 1-2 | " | |
| OCOC₅H₁₁ | 1-2 | " | |
| OCOC₁₈H₃₇ | 1-2 | " | |
| OH | 1-3 | o/ m/ p; m/ m/ p; o/p; m/ m | |
| F | 1-2 | o; p; o/p | |
| Cl | 1-2 | o; p; o/p | |
| CF₃ | 1 | o; m; p | |
| NO₂ | 1-3 | m/; m/ m; o/ o/ p | |
| NHCOR⁵ | 1 | p | |
| NHCOOR⁵ | 1 | p | |
| COR⁵ | 1-2 | o; p; o/p | |
| COOR⁵ | 1-2 | o; p; o/p | |
| CONR⁵R⁶ | 1-2 | o; p; o/p | |
| CN | 1 | p | |
| O=S(OR⁵)=O | 1 | p | |
| O=S(R⁵)=O | 1 | p | |
| O=S(NR⁵R⁶)=O | 1 | p | |
| R⁵OP(-OR⁶)=O | 1 | p | |

Die erfindungsgemäßen Chinoxalinderivate sind hervorragend geeignet als UV-Filtersubstanzen. Wie schon erwähnt, können die Chinoxaline synthetisch so gestaltet werden, dass durch Vorhandensein einer zusätzlichen chromophoren Gruppe UV-absorbierende Eigenschaften sowohl im UV-A- als auch im UV-B-Bereich resultieren. Somit kann ein Breitbandschutz erzielt werden. Ferner kann durch die Wahl der Substituenten die Löslichkeit der Substanzen in Wasser oder kosmetischen Ölen beeinflusst werden.

Im Folgenden (Tabelle 2) sind einige UV-A- und UV-B-Filtersubstanzen aufgelistet, mit denen die erfindungsgemäßen Chinoxaline vorzugsweise chemisch kombiniert werden können. Diese Auswahl soll in keiner Weise limitierend sein. Die Kombination kann durch übliche chemische Reaktionen erfolgen.

**Tabelle 2**

| **kombinierter UV-Filter** | **Beispiel** |
|---|---|
| 4-Aminobenzoesäure | s. Formel VIII |
| Dimethoxyphenylglyoxalsäure. | |
| 2,2',4,4'- Tetrahydroxybenzophenon | |
| 3,3,5-Trimethyl-cyclohexyl-salicylat | s. Formel IX |
| 2,2'-Dihydroxy-4-methoxy-benzophenon | |
| 5-Methyl-2-(1-methylethyl)-2-aminobenzoate | |
| 3-Imidazol-4-yl-acrylsäure und -ethylester | |
| 4-Isopropylbenzylsalicylat | |
| 1-(4'-Isopropylphenyl) -3-phenylpropan-1,3-dion | |
| 3-Benzyliden-bornan-2-on (3-Benzyliden-campher) | |
| 3-(4'-Methyl)benzyliden-bornan-2-on | s. Formel X |
| 3-(4'Trimethylammonium)-benzylidenbornan-2-on-methylsulfat | |
| 1-(4'-tert-Butylphenyl)-3-(4-methoxyphenyl)propan-1,3-dion (z.B. Eusolex® 9020) | s. Formel XI |
| 3-(4' Sulfo)benzyliden-bornan-2-on und Salze | |
| 2-Hydroxy-4-methoxy-benzophenon-5-sulfonsäure/ Na-Salz | |
| Salicylsäure-2-ethylhexylester | |
| 2-Hydroxy-4-methoxy-4'-methyl- benzophenon | |
| 2-Hydroxy-4-methoxy-benzophenon | |
| 4-Methoxy-zimtsäure2-isoamylester | |
| 4-Methoxy-zimtsäure2-ethylhexylester | |
| 4-Aminobenzoesäure-1-glycerylester | |

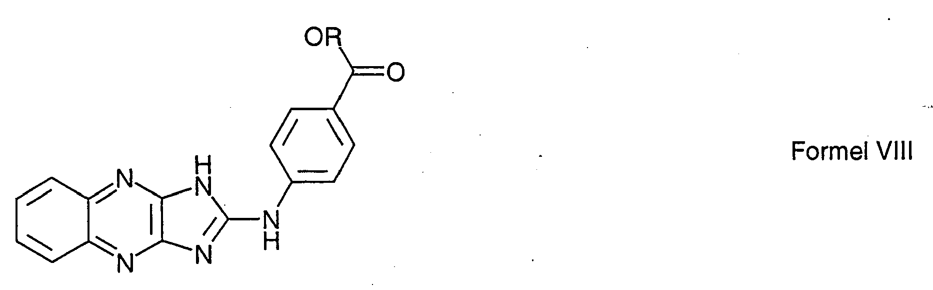

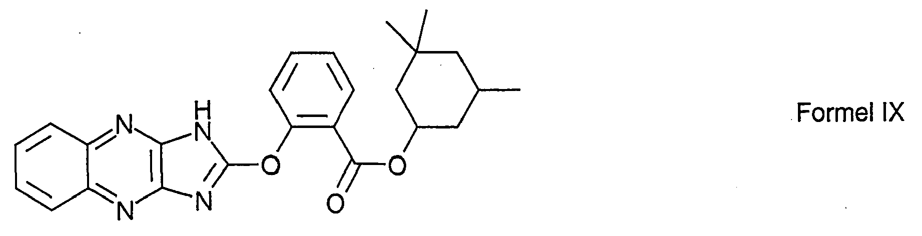

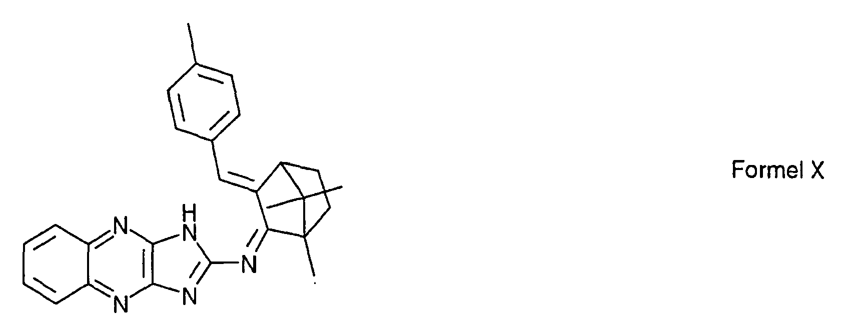

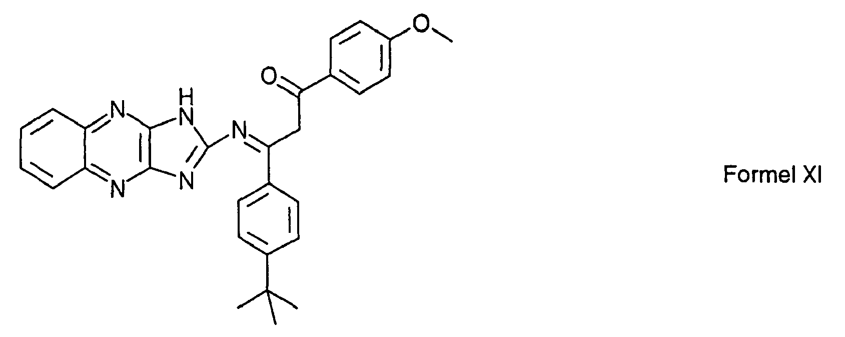

Daher ist es erfindungsgemäß besonders bevorzugt, wenn mindestens einer der Reste aus R bzw. R¹ bis R⁶ einem über eine geeignete funktionelle Gruppe (vorzugsweise eine Ester, Ether, Amin, Imin, Imid oder Amid-funktion) gebundenen UV-Filter aus Tabelle 2 entspricht.

Gegenstand der Erfindung sind auch die neuen Verbindungen gemäß Formel VIII 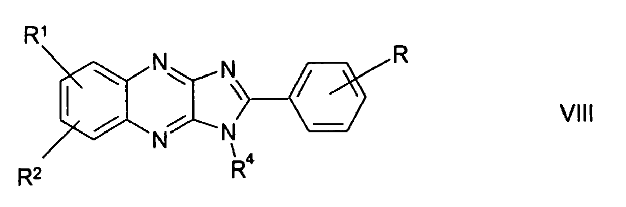 wobei,
R1 und R2 die in Anspruch 1 gegebene Bedeutung haben,
R4 steht für H oder einen verzweigten oder unverzweigten C1-20-Alkylrest, in dem gegebenenfalls ein oder mehrere H-Atome durch Sub ersetzt sein können,
Sub steht für H, Methyl, Ethyl oder Ethylhexyl und
R4 vorzugsweise steht für H, Methyl, Ethyl oder Ethylhexyl und
R im Phenylring steht für

| R | m | Position | 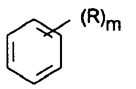 |
|---|---|---|---|
| CH₃ | 2- 3 | o/ m/ p ; m/ m/ p; o/p; m/ m | |
| C₂H₅ | 1-3 | " | |
| C_{3H7} | 1-3 | " | |
| ⁿC₄H₉ | 1-3 | " | |
| ^{t}C₄H₉ | 1-3 | " | |
| ⁱC₄H₉ | 1-3 | " | |
| C₅H₁₁ | 1-3 | " | |
| C₁₈H₃₇ | 1-3 | " | |
| (2'-Etyl)-Hexyl- | 1-3 | " | |
| OCH₃ | 3 | o/ m/ p ; m/ m/ p; o/p; m/ m | |
| OC₂H₅ | 1-3 | " | |
| OC₃H₇ | 1-3 | " | |
| OC₄H₉ | 1-3 | " | |
| OC₅H₁₁ | 1-3 | " | |
| OC₁₈H₃₇ | 1-3 | " | |
| OCOCH₃ | 1-2 | o; m; p; o/p | |
| OCOC₂H₅ | 1-2 | " | |
| OCOC₃H₇ | 1-2 | " | |
| OCOC₄H₉ | 1-2 | " | |
| OCOC₅H₁₁ | 1-2 | " | |
| OCOC₁₈H₃₇ | 1-2 | " | |
| OH | 2-3 | o/ m/ p ; m/ m/ p; m/ m | |
| F | 1-2 | o; p; o/p | |
| Cl | 1-2 | o; p; o/p | |
| CF₃ | 1 | o; m; p | |
| NO₂ | 2-3 | m/ m; o/ o/ p | |
| NHCOR⁵ | 1 | P | |
| NHCOOR⁵ | 1 | P | |
| COR⁵ | 1-2 | o; p; o/p | |
| COOR⁵ | 1-2 | o; p; o/p | |
| CONR⁵R⁶ | 1-2 | o; p; o/p | |
| CN | 1 | P | |
| O=S(OR⁵)=O | 1 | P | |
| O=S(R⁵)=O | 1 | P | |
| O=S(NR⁵R⁶)=O | 1 | P | |
| R⁵OP(-OR⁶)=O | 1 | P | |

Die erfindungsgemäßen Chinoxalinderivate gemäß Formel IV können ausgehend von 2,3-Dichlorchinoxalinen, die entsprechend der Zielverbindung mit R¹ und R² substituiert sind, hergestellt werden. Ein derartiges Verfahren ist daher ein weiterer Gegenstand der vorliegenden Erfindung.

In einer bevorzugten Variante wird dabei in einem ersten Reaktionsschritt ein Chloratom im 2,3-Dichlorchinoxalin gegen eine Aminofunktion ausgetauscht. In einem weiteren Reaktionsschritt wird vorzugsweise das zweite Chloratom gegen -NH-R⁴ ausgetauscht.

In einer anderen ebenfalls bevorzugten Verfahrensvariante wird in einem ersten Reaktionsschritt ein Chloratom im 2,3-Dichlorchinoxalin gegen -NH-R⁴ ausgetauscht und in einem zweiten Reaktionsschritt das zweite Chloratom gegen eine Aminofunktion.

Der Ringschluß zum erfindungsgemäßen Imidazochinoxalin erfolgt dann in beiden Fällen bevorzugt durch Umsetzung mit einem geeignet substituierten Benzaldehyd-Derivat.

Die Wahl geeigneter Reaktionsbedingungen für diese Umsetzungen bereitet dem Fachmann keinerlei Schwierigkeiten. Geeignete Bedingungen werden für ein exemplarisches Beispiel (Herstellung von ist 1-(2-Ethyl-hexyl)-2-phenylimidazo[3,4-b]chinoxalin) in Beipiel H angegeben.

Die Chinoxalinderivate können auch, falls gewünscht, mit beliebigen UV-Filtersubstanzen kombiniert werden, was zu einer Verbesserung der protektiven Leistung (SPF-Boost) durch synergistische Effekte führt. Im Folgenden sind einige UV-A- und UV-B-Filtersubstanzen aufgelistet, mit denen die erfindungsgemäßen Chinoxaline vorzugsweise kombiniert werden können. Diese Auswahl soll in keiner Weise limitierend sein. Die Kombination kann neben der direkten chemischen Reaktion (wie oben in Tabelle 2 gezeigt) natürlich auch durch physikalische Kombination mit UV-Filtern erfolgen. Prinzipiell kommen alle UV-Filter für eine Kombination in Frage. Besonders bevorzugt sind solche UV-Filter, deren physiologische Unbedenklichkeit bereits nachgewiesen ist. Sowohl für UV-A wie auch UV-B-Filter gibt es aus der Fachliteratur bekannte Substanzen, z.B.

Benzylidenkampferderivate wie
- 3-(4'-Methylbenzyliden)-dl-kampfer (z.B. Eusolex^{®} 6300),
- 3-Benzylidenkampfer (z.B. Mexoryl^{®} SD),
- Polymere von N-{(2 und 4)-[(2-oxoborn-3-yliden)methyl]benzyl}-acrylamid (z.B. Mexoryl^{®} SW),
- N,N,N-Trimethyl-4-(2-oxoborn-3-ylidenmethyl)anilinium methylsulfat (z.B. Mexoryl^{®} SK) oder
- α-(2-Oxoborn-3-yliden)toluol-4-sulfonsäure (z.B. Mexoryl^{®} SL),

Benzoyl- oder Dibenzoylmethane wie
- 1 -(4-tert-Butylphenyl)-3-(4-methoxyphenyl)propan-1 ,3-dion (z.B. Eusolex® 9020) oder
- 4-Isopropyldibenzoylmethan (z.B. Eusolex® 8020),

- Benzophenone wie
   - 2-Hydroxy-4-methoxybenzophenon (z.B. Eusolex® 4360) oder
   - 2-Hydroxy-4-methoxybenzophenon-5-sulfonsäure und ihr Natriumsalz (z.B. Uvinul® MS-40),
- Methoxyzimtsäureester wie
   - Methoxyzimtsäureoctylester (z.B. Eusolex® 2292),
   - 4-Methoxyzimtsäureisopentylester, z.B. als Gemisch der Isomere (z.B. Neo Heliopan® E 1000),
- Salicylatderivate wie
   - 2-Ethylhexylsalicylat (z.B. Eusolex® OS),
   - 4-lsopropylbenzylsalicylat (z.B. Megasol®) oder
   - 3,3,5-Trimethylcyclohexylsalicylat (z.B. Eusolex® HMS),
   - 4-Aminobenzoesäure und Derivate wie
   - 4-Aminobenzoesäure,
   - 4-(Dimethylamino)benzoesäure-2-ethylhexylester (z.B. Eusolex® 6007),
   - ethoxylierter 4-Aminobenzoesäureethylester (z.B. Uvinul® P25),
und weitere Substanzen wie
- 2-Cyano-3,3-diphenylacrylsäure-2-ethylhexylester (z.B. Eusolex^{®} OCR),
- 2-Phenylbenzimidazol-5-sulfonsäure sowie ihre Kalium-, Natrium- und Triethanolaminsalze (z.B. Eusolex^{®} 232),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl^{®} SX) und
- 2,4,6-Trianilino-(p-carbo-2'-ethylhexyl-1'-oxi)-1,3,5-triazin (z.B. Uvinul^{®} T 150).

Die in der Liste aufgeführten Verbindungen sind nur als Beispiele aufzufassen. Selbstverständlich können auch andere UV-Filter verwendet werden.
Diese organischen UV-Filter werden in der Regel in einer Menge von 0,5 bis 10 Gewichtsprozent, vorzugsweise 1 - 8 %, in kosmetische Zubereitungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. UV-Asorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethyl butyl)phenol) (CAS-Nr. 103 597-45-1)
- 2,2'-(1,4-Phenylen)bis)-1H-benzimidazol-4,6-disulfonsäure, Mononatriumsalz) (CAS-Nr. 180 898-37-7) und
- 2,2'-(1,4-Phenylen)bis)-1H-benzimidazol-5,5'-monosulfonsäure, Mononatriumsalz
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

Diese organischen UV-Filter werden in der Regel in einer Menge von 0,5 bis 20 Gewichtsprozent, vorzugsweise 1 - 15 %, in kosmetische Zubereitungen eingearbeitet. In erfindungsgemäßen Formulierungen liegen sie dabei üblicherweise in Gewichtsverhältnissen von 15:1 bis 1:15, vorzugsweise von 10:1 bis 1:10 und insbesondere bevorzugt von 5:1 bis 1:5 zu den Chinoxalinderivaten der Formel Ivor.

Bevorzugte Verbindungen mit UV-filternden Eigenschaften sind 3-(4'-Methylbenzyliden)-dl-kampfer, 1-(4-tert-Butylphenyl)-3-(4-methoxyphenyl)-propan-1,3-dion, 4-Isopropyldibenzoylmethan, 2-Hydroxy-4-methoxybenzophenon, Methoxyzimtsäureoctylester, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Als anorganische UV-Filter sind beispielsweise solche aus der Gruppe der Titandioxide wie z.B. gecoatetes Titandioxid (z.B. Eusolex^{®} T-2000, Eusolex®T-AQUA), Zinkoxide (z.B. Sachtotec^{®}), Eisenoxide oder auch Ceroxide denkbar. Titanoxid und Zinkoxid liegen dabei vorzugsweise als mikronisierte anorganische Pigmente vor. Diese anorganischen UV-Filter werden in der Regel in einer Menge von 0,5 bis 20 Gewichtsprozent, vorzugsweise 2 - 10 %, in kosmetische Zubereitungen eingearbeitet. In erfindungsgemäßen Formulierungen liegen sie dabei üblicherweise in Gewichtsverhältnissen von 25:1 bis 1:25, vorzugsweise von 10:1 bis 1:10 und insbesondere bevorzugt von 5:1 bis 1:5 zu den Chinoxalinderivaten der Formel I vor.

Die erfindungsgemäße Verwendung der Chinoxalinderivate hat einen weiteren, interessanten und vorteilhaften Aspekt. Die Kombination mit anderen UV-Filtern führt oftmals zur einer Photostabilisierung dieser UV-Filter durch das Chinoxalinderivat.

Es ist ja bekannt, dass einige UV-Filter, die an sich vorteilhafte . Lichtschutzfiltereigenschaften besitzen, den großen Nachteil einer gewissen Instabilität gegenüber UV-Strahlung haben.

Beispielsweise sind die Dibenzoylmethanderivate, wie das Eusolex 9020 (4-t-Butyl-4'-methoxy-dibenzoylmethane), solche Substanzen, die der photochemischen Zersetzung ausgesetzt sind. Die photochemischen Zersetzung dieser Verbindungsklasse folgt einer Norrish-Typ-I-Acylspaltung. Die dabei entstehenden Reaktionsprodukte stehen als Lichtschutzfiltersubstanzen nicht mehr zu Verfügung. Auch wenn schon einige Lösungsvorschläge im Stand der Technik aufgezeigt wurden, besteht dennoch stets ein Bedarf an einfachen und effektiven Wegen, dieser photolytischen Zersetzung wirksam zu begegnen.

Dafür eignen sich die hier beschriebenen Chinoxalinderivate auf hervorragende Weise, insbesondere die Kombination mit Eusolex 9020 führt zu einer stark verbesserten Photostabilisierung der Substanz.

Dabei können in bevorzugten Ausführungsformen der Erfindung sowohl die erfindungsgemäßen Verbindungen gemäß Formel I als auch die üblichen oben aufgeführten UV-Filter in Kapseln eingeschlossen vorliegen. Insbesondere bevorzugt ist es solche UV-Filter zu verkapseln, die entweder in der Formulierung nicht Lagerstabil sind oder dazu neigen in der Anwendung die Haut zu penetrieren, die Verkapselung kann dabei nach üblichen aus der Literatur bekannten Methoden erfolgen. Als Kapselmaterial eignen sich dabei organische und anorganische Polymere. Insbesondere bevorzugt ist es, die UV-Filter, und insbesondere auch die erfindungsgemäßen Chinoxalinderivate nach Formel I in Oxid-Kapseln, vorzugsweise Silica-Kapslen einzuschließen, die vorzugsweise in einem Sol-Gel-Prozeß hergestellt werden, wie er in der internationalen Anmeldung WO 00/09652 beschrieben ist.

Erfindungsgemäß können die hier beschriebenen UV-Lichtschutzfilter jeweils für sich allein oder natürlich auch in Kombination, was bevorzugt ist, in Sonnenschutzmitteln verwendet werden. Sie können mit UV-B/A-Chromophoren, z.B. allen weltweit zugelassenen und bekannten Filtern kombiniert werden zur Verbesserung der protektiven Leistung (SPF-Boost) durch synergistische Effekte. Sie sind vorzugsweise in Kombination sowohl mit anorganischen als auch mit organischen UV-A- und UV-B-Filtern oder deren Gemischen einsetzbar.

Durch Kombination von einer oder mehrerer Verbindungen der Formel I mit weiteren UV-Filtern kann die Schutzwirkung gegen schädliche Einwirkungen der UV-Strahlung optimiert werden.

Ferner ist es möglich und vorteilhaft, die erfindungsgemäßen Zubereitungen mit Antioxidationsmitteln zu kombinieren. Eine solche Kombination zeigt dann sowohl Schutzwirkung als Antioxidationsmittel als auch vor Verbrennungen durch UV-Strahlung. Somit kann man auch eine schützende Wirkung gegen oxidativen Stress bzw. gegen die Einwirkung von Radikalen erzielen.

Gegenstand der Erfindung ist daher auch die Verwendung einer Verbindung der Formel I gemäß Anspruch 1 in Kombination mit Antioxidationsmitteln in kosmetischen oder pharmazeutischen Zubereitungen.

Gegenstand der vorliegenden Erfindung sind daher auch kosmetische und pharmazeutische Zubereitungen, die eine oder mehrere der Verbindungen der Formel I, gegebenenfalls in Kombination mit weiteren Lichtschutzmitteln oder Antioxidationsmitteln, enthalten.

Gegenstand der Erfindung ist auch ein Verfahren zum Schutz der Haut und natürlicher oder sensibilisierter Haare vor Sonnenstrahlen, wobei auf die Haut oder die Haare eine wirksame Menge mindestens einer Verbindung der Formel I in einer kosmetische Zubereitung aufgetragen wird. Mit "sensibilisierten Haaren" sind Haare gemeint, welche beispielsweise einer Dauerwellenbehandlung, einem Färbe- oder Entfärbeprozess unterzogen worden sind.

Die erfindungsgemäßen Filter zum Schutz vor UV-A und UV-B-Strahlung können jeweils in Konzentrationen von 0,1 bis 20 Gew. %, vorzugsweise 1 bis 15 Gew.-%, in kosmetische Zubereitungen eingearbeitet werden. Es ist auf diese Weise möglich Zubereitungen herzustellen, in denen bis zu 100 % der eingesetzten Lichtschutzfilter die hier beschriebenen UV-Filter sind. Es handelt sich hierbei um Substanzen, die in Wasser und Ölen, je nach Substituenten am Gerüst, in einfacher Weise gelöst, dispergiert oder emulgiert werden.

Die erfindungsgemäßen Zubereitungen können darüber hinaus weitere übliche hautschonende oder hautpflegende Wirkstoffe enthalten. Dies können prinzipiell alle dem Fachmann bekannten Wirkstoffe sein.

Besonders bevorzugte Wirkstoffe sind Pyrimidincarbönsäuren und/oder Aryloxime, sowie Coumaranonderivate.

Pyrimidincarbonsäuren kommen in halophilen Mikroorganismen vor und spielen bei der Osmoregulation dieser Organismen eine Rolle (*E.* A. *Galinski et al., Eur. J. Biochem., 149 (1985) Seite 135-139*). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden.

Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel eingesetzt, 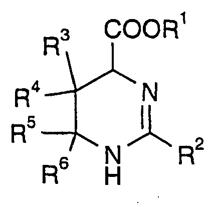 worin R¹ ein Rest H oder C1-8-Alkyl, R² ein Rest H oder C1-4-Alkyl und R³, R⁴, R⁵ sowie R⁶ jeweils unabhängig voneinander ein Rest aus der Gruppe H, OH, NH₂ und C1-4-Alkyl sind. Bevorzugt werden Pyrimidincarbonsäuren eingesetzt, bei denen R² eine Methyl- oder eine Ethylgruppe ist und R¹ bzw. R⁵ und R⁶ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Unter Coumaranonderivaten, die in erfindungsgemäßen Zubereitungen vorteilhaft enthalten sein können, werden Verbindungen der Formel 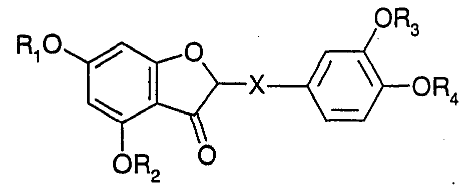 wobei -X- steht für eine Einfachbindung, -CH₂-, =CH-, -C(O)-, =C(OR⁵)-,-C(NR⁵)-, -CH(NR⁵R⁶)-, -CH(OR⁵)- und R¹, R², R³, R⁴, R⁵, und R⁶ gleich oder verschieden sein können, und unabhängig voneinander stehen für
- H
- geradkettige oder verzweigte C₁- bis C₁₂-Alkyl und/oder Alkylcarbonylgruppen,
- geradkettige oder verzweigte C₃- bis C₁₂-Alkenyl und/oder -Alkenylcarbonylgruppen,
- geradkettige oder verzweigte C₁- bis C₁₂-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann,
- C₃- bis C₁₀-Cycloalkyl- und/oder Cycloalkylcarbonylgruppen und C₃- bis C₁₂-Cycloalkenyl- und/oder Cycloalkenylcarbonylgruppen, wobei die Ringe jeweils auch durch -(CH₂)ₙ-Gruppen mit n = 1 bis 3 überbrückt sein können,
- Aryl- und/oder Arylcarbonylgruppen,
- Heteroaryl- und/oder Heteroarylcarbonylgruppen, wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,
- Mono- und/oder Oligoglycosylreste, 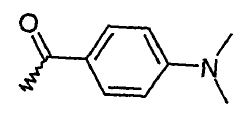 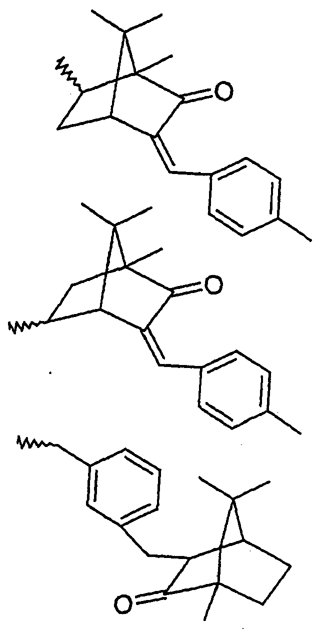 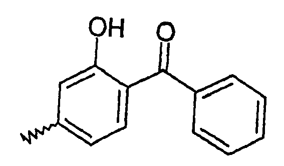 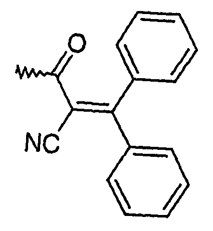 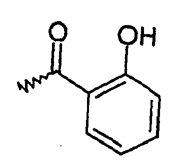 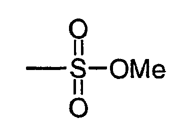 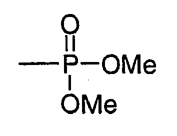 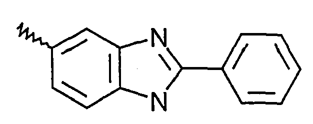 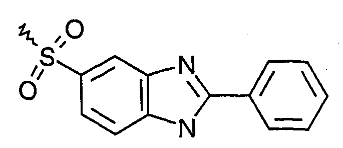 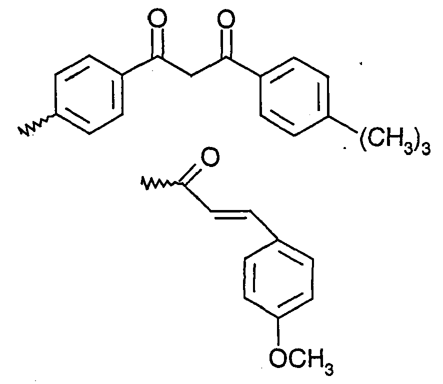 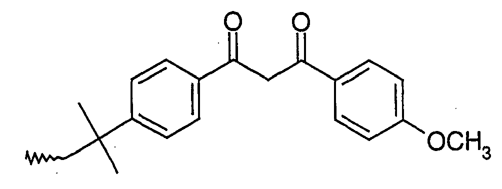
wobei Me steht für ein Proton oder ein Alkalimetallion, insbesondere ein Natrium- oder Kaliumion.

Die Reste können also als Ether oder als Ester an den Grundkörper gebunden sein. Derartige Verbindungen werden in der Deutschen Patentanmeldung DE 10003785.2 beschrieben. Zusammensetzungen, die derartige Coumaranonderivate enthalten, wirken besonders hautschonend, die diese Verbindungen antioxidante und radikalfangende Wirkung zeigen.

Sehr gute Eigenschaften besitzt der Grundkörper 4,6,3',4'-Tetrahydroxybenzylcoumaranon-3. Dies entspricht der oben stehenden Formel, wobei X = -CH₂- und R¹ = R² = R³ = R⁴ = H ist. Die Löslichkeit dieser Verbindung in Wasser kann verbessert werden, indem beispielsweise die Reste R¹, R², R³ und R⁴ als Sulfat- oder Phosphatgruppen gewählt werden. Besonders geeignet ist ein Gemisch aus Mono-, Di- und Trisulfat, das im weiteren als "sulfatisiertes Coumaranon" bezeichnet wird. Besonders hervorgehoben ist das Trisulfat (X = -CH₂-; R¹ = R³ = R⁴ = SO₃Me, R² = H), das durch die folgende Formel dargestellt wird.

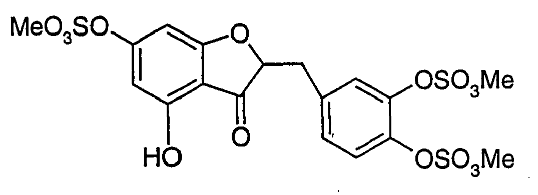

Alle Verbindungen oder Komponenten, die in den Zubereitungen verwendet werden können, sind entweder bekannt und käuflich erwerbbar oder können nach bekannten Verfahren synthetisiert werden.

Die Lichtschutzmittel enthaltenden kosmetischen und pharmazeutischen Zubereitungen sind in der Regel auf der Basis eines Vehikels, welches mindestens eine Ölphase enthält. Es sind aber auch Zubereitungen allein auf wässriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können somit in flüssiger, pastöser oder fester Form vorliegen, beispielsweise als Wasser-in-Öl-Cremes, Öl-in-Wasser-Cremes und -Lotionen, W/O/W-Systeme oder O/W/O-Systeme, Aerosol-Schaumcremes, Gele, Öle, Fettstifte, Puder, Sprays oder alkoholischwässrige Lotionen. Ferner können sie auch als mikronisierte Systeme oder als PIT-(Phasen-Inversion-Temperatur)-Emulsionen formuliert sein.

Übliche Ölkomponenten in der Kosmetik sind beispielsweise Paraffinöl, Glycerylstearat, Isopropylmyristat, Düsorpopyladipat, 2-Ethylhexansäureacetylstearylester, hydriertes Polyisobuten, Vaseline, Caprylsäure/Caprinsäure-Triglyceride, mikrokristallines Wachs, Lanolin, Mineralöle, Mineralwachse, Ester von Fettsäuren mit Akoholen niedriger C-Zahl, z.B. mit Isopropanol, Propylenglykol oder Glycerin, Alkylbenzoate, Silikonöle wie Dimethylpolysiloxane, Diethylpolysiloxane, Diphenylpolysiloxane und Stearinsäure.

Die Zubereitungen können kosmetische Hilfsstoffe enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Fette und Wachse, Lanolin, Treibmittel, Stabilisatoren, Antioxidantien; Bakterizide, Farbstoffe und/oder Pigmente, welche das Mittel selbst oder die Haut färben, und andere in der Kosmetik gewöhnlich verwendete Ingredienzien.

Als Emulgatoren kommen vorzugsweise bekannte W/O- und daneben auch O/W-Emulgatoren wie etwas Polyglycerinester, Sorbitanester oder teilveresterte Gylceride in Betracht. Typische Beispiele für Fette sind Glyceride; als Wachse sind u.a. Bienenwachs, Carnaubawachs, Paraffinwachs oder Mikrowachse, gegebenenfalls in Kombination mit hydrophilen Wachsen zu nennen. Als Stabilisatoren können Metallsalze von Fettsäuren wie z.B: Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Ferner können auch Pflanzenextrakte, Eiweißhydrolysate und Vitaminkomplexe zugesetzt werden.

Als Antioxidantien können beispielsweise Aminosäuren, Imidazole, Peptide, Carotinoide, α-Hydroxysäuren, ungesättigte Fettsäuren, Vitamin A, C und/oder E und geeignete Derivate dieser genannten Stoffe verwendet werden.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate .(z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO4), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

In erfindungsgemäßen Formulierungen liegen die Chinoxalinderivate der Formel I zu den Antioxidantien dabei üblicherweise in Gewichtsverhältnissen von 10.000:1 bis 1:5, vorzugsweise von 500:1 bis 1:2 und insbesondere bevorzugt von 50:1 bis 1:1 vor.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die wässrige Phase der erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol Ethylenglykolmonoethyl- oder -monobutylether oder analoge Produkte, ferner Alkohole wie Ethanol, Isopropanol, 1,2-Propandiol sowie insbesondere ein oder mehrere Verdickungsmittel, wie z.B. Siliciumdioxid, Aluminiumsilikate, Polysaccharide bzw. deren Derivate, z.B. Hyaluronsäure, Xanthangummi, Hydroxypropylemethylcellulose, oder ein Polyacrylat aus der Gruppe der sogenannten Carbopole.

Gebräuchliche Filmbildner sind beispielsweise Hydrocolloide wie Chitosan, mikrokristallines Chitosan oder quaternäres Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösungen, p-Hydroxybenzoat oder Sorbinsäure. Als Perlglanzmittel kommen beispielsweise Glycoldistearinsäureester wie Ethylenglydoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentrationen von 0,001 bis 0,1 Gew.-%, bezogen auf die gesamte Mischung, eingesetzt.

Gegebenenfalls können die erfindungsgemäßen Sonnenschutzmittel auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Zubereitungen geeignet sind, eingesetzt werden. Solche können sowohl pflanzliche Extrakte als auch synthetische Selbstbräuner, wie z.B. Dihydroxyaceton oder α-Ketole, sein.

Soll das erfindungsgemäße Mittel natürliche oder sensibilisierte Haare vor Sonneneinstrahlung schützen, so kann es als Shampoo, Lotion, Gel oder Emulsion zum Ausspülen vorliegen, wobei die jeweilige Zubereitung vor oder nach dem Schampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Haarspray, Aerosol-Schaumcreme, Dauerwellmittel, Färbe- oder Entfärbemittel der Haare vor. Dieses Mittel kann außer den erfindungsgemäßen Lichtschutzfiltern (VIS- und/oder IR-Filter) oder der erfindungsgemäßen Kombination von Lichtschutzfiltern verschiedene, in diesem Mitteltyp verwendete AdjUV-Anzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Die erfindungsgemäßen Lichtschutzfilter lassen sich direkt ohne weitere vorbereitende Maßnahmen in kosmetischen Zubereitungen einarbeiten.

Diese Substanzen bieten ferner den großen Vorteil, keine toxischen oder allergischen Reaktionen gegenüber der Haut zu zeigen.

Vorteilhaft können die Lichtschutz Zubereitungen erfindungsgemäß, wie vorstehend schon beschrieben, weitere UV-Filtersubstanzen enthalten, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Weiterhin können die erfindungsgemäßen Zubereitungen auch als pharmazeutische Mittel zur vorbeugenden Behandlung von Entzündungen und Allergien der Haut sowie auch in bestimmten Fällen zur Verhütung bestimmter Krebsarten verwendet werden. Das erfindungsgemäße pharmazeutische Mittel kann oral oder topisch verabreicht werden. Für die orale Verabreichung liegt die pharmazeutische Zubereitung in Form von u.a. Pastillen, Gelatinekapseln, Dragees, als Sirup, Lösung, Emulsion oder Suspension vor. Die topische Anwendung erfolgt beispielsweise als Salbe, Creme, Gel, Spray, Lösung oder Lotion.

Die erfindungsgemäßen kosmetischen und pharmazeutischen Zubereitungen können mit Hilfe von Techniken hergestellt werden, die dem Fachmann wohl bekannt sind.

Ebenso positiv sind die Eigenschaften von Verbindungen der Formel 1 zu werten für eine Verwendung in Nahrungsmitteln, als Nahrungsergänzungsmittel,als diätetisches Mittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren der erfindungsgemäßen Verbindungen angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel I angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren der erfindungsgemäßen Verbindungen angereichert zu werden, beispielsweise Multivitaminpräparate, Mineralstoffmischungen oder gezuckerter Saft. Als weitere Beispiele für Nahrungsmittel seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formeln I angereichert werden können, werden vorzugsweise oral angewendet, z.B. in Form von Speisen, Pillen, Tabletten, Kapseln, Pulver, Sirup, Lösungen oder Suspensionen.

Diese angereicherten erfindungsgemäßen Nahrungsmittel können mit Hilfe von Techniken hergestellt werden, die dem Fachmann wohl bekannt sind.

Auch ohne weitere Ausführungen wird davon ausgegangen, dass ein Fachmann die obige Beschreibung im weitesten Umfang nutzen kann. Die bevorzugten Ausführungsformen sind deswegen lediglich als beschreibende, keineswegs als in irgendeine Weise limitierende Offenbarung aufzufassen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. das Gesamtgewicht der Zubereitungen bezogen.

Die vollständige Offenbarung aller vor- und nachstehend aufgeführten Anmeldungen und Veröffentlichungen sind durch Bezugnahme in diese Anmeldung eingeführt.

### Herstellungsbeispiele

### Beispiel A: Synthese von 1H-Pyrazolo [3,4-b]chinoxalinen (A und B)

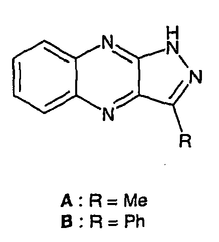

Herstellung: In THF werden 320 mg des 2-Carbonyl-3-chlor-chinoxalins (1.55 mmol) und Hydrazin-Hydrat (194 mg, 3.88 mmol) 72 h zum Sieden erhitzt. Das Lösungsmittel wird abdestilliert, und zu dem Rückstand wird Wasser (50 ml) und gesättigte Natriumhydrogencarbonat-Lösung (10 ml) gegebnen. Es wird mit Chloroform (3 x 100 ml) extrahiert und die vereinigten organischen Phasen über Magnesiumsulfat getrocknet. Das Lösungsmittel wird abdestilliert und nach der Umkristallisation des Rückstands aus Cyclohexan/tert-Butylmethylether kann das 3-Methyl-1*H*-pyrazolo-chinoxalin und das 3-Phenyl-1*H*-pyrazolo-chinoxalin in einer Ausbeute von 80 % bzw. 97 %, gewonnen werden.

### Charakterisierung von 3-Methyl-1H-pyrazolo[3,4-b]-chinoxalin (A)

Schmelzpunkt: 225°C
300 MHz-¹H-NMR (CDCl₃):

| | | |
|---|---|---|
| δ= | 13.55 | (s br., 1H, -NH, mit D₂O austauschbar) |
| | 8.19-7.71 | (m, 4 H, Hetaryl arom.) |
| | 2.66 | (s, 3H, CH₃) |

75 MHz-¹³C-NMR (CDCl₃):

| | | |
|---|---|---|
| δ= | 143.76 | (quart. C) |
| | 142.20 | (quart. C) |
| | 140.94 | (quart. C) |
| | 139.79 | (quart. C) |
| | 135.59 | (quart. C) |
| | 130.46 | (tert. C) |
| | 129.80 | (tert. C) |
| | 128.16 | (tert. C) |
| | 128.24 | (tert. C) |
| | 11.43 | (-CH₃) |
| MS(70eV):m/z(%)= | 185 (13) [M⁺+1], 184 (100) [M⁺], 183 (12), 143 (40), 116 (20), 102(10), 90 (10) | |

C₁₀H₈N₄ (184.20 g/mol):

| | | | |
|---|---|---|---|
| Ber.[%] | C 65.21 | H 4.38 | N 30.42 |
| Gef.[%] | C 64.92 | H 4.36 | N 30.23 |

### Charakterisierung von 3-Phenyl-1H-pyrazolo[3,4-b]-chinoxalin (B)

Schmelzpunkt: 251 °C
300 MHz-¹H-NMR (CDCl₃):

| | | |
|---|---|---|
| δ= | 14.35 | (s br., 1H, NH, mit D₂O austauschbar) |
| | 8.63 - 7.35 | (m, 4 H, Hetaryl, 5 H, Phenyl) |
| | 8.31 - 8.63 | (m_{c}, 4H, Hetaryl arom.) |
| | 7.35 - 7.89 | (m_{c}, 5H, arom.) |

75 MHz-¹³C-NMR (CDCl₃):

| | | |
|---|---|---|
| δ= | 144.27 | (quart. C) |
| | 141.61 | (quart. C) |
| | 140.88 | (quart. C) |
| | 140,50 | (quart. C) |
| | 134.62 | (quart. C) |
| | 133.64 | (quart. C) |
| | 131.00 | (quart. C) |
| | 130.11 | (quart. C) |
| | 128.83 | (3 x tert. C) |
| | 128.40 | (tert. C) |
| | 128.01 | (tert. C) |
| | 126.24 | (2 x tert. C) |
| MS(70eV):m/z (%)= | 247 (18) [M++1], 246 (100) [M+], 245 (15), 219 (17), 143 (18), 116(17), 89 (10), 58 (10) | |

C₁₅H₁₀N₄ (246.27 g/mol):

| | | | |
|---|---|---|---|
| Ber.[%] | C 73.16 | H 4.09 | N 22.75 |
| Gef.[%] | C 73.22 | H 4.18 | N 22.58 |

### Beispiel B: Synthese der N-Aryl=pyrrolo[2,3-b]-chinoxaline

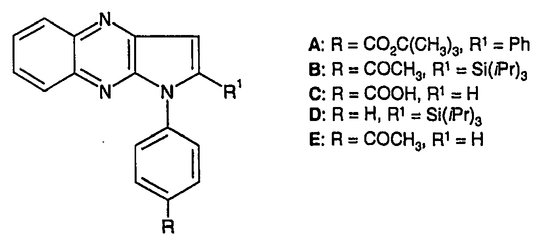

Herstellung: Zu einer Lösung von 1,00 mmol des 2-Alkinyl-3-chlor-chinoxalins in einem polaren Lösungsmittel (wasserfrei) (R¹=H, Ph; Si(iPr)₃ : THF) werden 1.05 mmol des aromatischen Amins gegeben und 2-12 h unter Rückfluß erhitzt. Der während der Reaktion, bzw. durch Einengen der Lösung ausgefallene Feststoff, wird abfiltriert und durch Umkristallisation in Ethanol/Essigester, oder durch Säulenchromatographie an Kieselgel, mit Cyclohexan/Essigester (1:1) aufgereinigt. Die Pyrrolo-chinoxaline werden in Ausbeuten von 53-88% erhalten.

### Charakterisierung von N-[4(t-Butyloxycarbonyl)phenyl]-2-phenyl[2,3-b]pyrrolochinoxalin (A)

IR (KBr):

| | | |
|---|---|---|
| Wellenzahl [cm⁻¹] = | 3132 | (ν (N-R), NR₂) |
| | 3065 | (ν (C-H), Ph) |
| | 2973 | (ν (C-H), *t*-Butyl) |
| | 1710 | (ν (C=O), CO₂R) |
| | 1617 | (ν(C=C), Ph) |
| | 1499 | (ν(C=C), Ph) |
| | 1341 | ((Gerüst), Ph) |
| | 1308 | ((Gerüst), Ph) |
| | 792 | (γ (C-H), Ph) |
| | 762 | (γ (C-H), Ph) |

300 MHz-¹H-NMR (CDCl₃):

| | | |
|---|---|---|
| δ = | 8.17-8.20 | (m, 1H, Hetaryl arom.) |
| | 8.03-8.08 | (m, 1H, Hetaryl arom.) |
| | 7.99-8.02 | (m, 2H, arom.) |
| | 7.73-7.78 | (m, 2H, Hetaryl arom.) |
| | 7.54-7.57 | (m, 2H, arom.) |
| | 7.43-7.49 | (m, 5H, arom.) |
| | 7.30 | (s, 1H, Hetaryl arom.) |
| | 1.58 | (s, 9H, C₄H₉) |

75 MHz-¹³C-NMR (CDCl₃):

| | | |
|---|---|---|
| δ= | 166.64 | (C_{quart}, CO₂R) |
| | 150.89 | (C_{quart}, arom.) |
| | 143,71 | (C_{quart}, arom.) |
| | 142.65 | (C_{quart}, arom.) |
| | 140.80 | (C_{quart}, arom.) |
| | 139.59 | (C_{quart}, arom.) |
| | 138.50 | (C_{quart}, arom.) |
| | 130.11 | (C-H, arom.) |
| | 129.83 | (C-H, arom.) |
| | 129.58 | (C-H, arom.) |
| | 129.01 | (C-H, arom.) |
| | 128.68 | (C-H, arom.) |
| | 128.44 | (C-H, arom.) |
| | 128.07 | (C-H, arom.) |
| | 127.98 | (C-H, arom.) |
| | 127.24 | (C-H, arom.) |
| | 102.06 | (C-3, Pyrrol) |
| | 51.43 | (C(CH₃)₃) |
| | 27.67 | (C(CH₃)₃) |
| MS(70eV):m/z(%)= | 422 (16) [M⁺+1], 421 (58) [M⁺], 406 (2), 360 (100), 349 (9), 321 (37), 295 (2), 218 (1), 191 (2), 174 (4), 137 (2), 128 (3), 120 (5), 102 (4), 91 (2), 76 (4) | |

### Charakterisierung von N-[4-(Acetyl)-phenyl]-2-(triisopropyl)silyl-pyrrolo[2,3-b] chinoxalin (B)

IR (KBr):

| | | |
|---|---|---|
| Wellenzahl [cm⁻¹] = | 3183 | (ν (N-R), NR2) |
| | 3046 | (ν (C-H), Ph) |
| | 1697 | (ν (C=O), COCH3) |
| | 1643 | (ν(C=C), Ph) |
| | 1597 | (ν(C=C), Ph) |
| | 1583 | (ν(C=C), Ph) |
| | 1347 | ((Gerüst), Ph) |
| | 1282 | ((Gerüst), Ph) |
| | 841 | (γ (Si-C), SiR3) |
| | 755 | (γ (C-H), Ph) |

300 MHz-¹H-NMR (CDCl3):

| | | |
|---|---|---|
| δ = | 8.32 | (m, 1H, Hetaryl arom.) |
| | 8.05-8.08 | (m, 1H, Hetaryl arom.) |
| | 7.98-8.01 | (m, 2H', arom.) |
| | 7.71-7.77 | (m, 2H, Hetaryl arom.) |
| | 7.56-7.59 | (m, 2H, arom.) |
| | 7.25 | (s, 1H, Hetaryl arom.) |
| | 2.14 | (s, 3H, CH3) |
| | 1.35 | (sep, 3H, CH) |
| | 1.13 | (s, 9H, CH3) |
| | 1.09 | (d, 9 H, CH3) |
| MS(70eV):m/z (%)= | 443 (3) [M+], 373 (2), 344 (1), 303 (43), 301 (100), 286 (1), 261 (63), 259 (25), 245 (7), 231 (68), 217 (7), 195 (11), 181 (8), 153 (7), 122 (19), 103 (43), 93 (13), 79 (6) | |

HRMS (hochaufgelöste Masse) (EI) (C₂₇H₃₃N₃O Si) [M⁺]
Ber. für: 443, 2447
Gef. für: 443, 2446

### Charakterisierung von 4-[N-(Pyrrolo[2,3-b] chinoxalin)] benzoesäure (C)

IR(KBr):

| | | |
|---|---|---|
| Wellenzahl [cm⁻¹] = | 3133 | (ν (N-R), NR₂) |
| | 3056 | (ν (C-H), Ph) |
| | 1710 | (γ (C=O)) |
| | 1611 | (γ (C=C), Ph) |
| | 1493 | (γ (C=C), Ph) |
| | 1414 | ((Gerüst), Ph) |
| | 1348 | ((Gerüst), Ph) |
| | 762 | (γ (C-H), Ph) |
| MS(70eV):*m*/*z* (%)= | 365 [M⁺] (100), 364 (61), 319 (37), 262 (7), 217 (2), 183 (6), 102 (9), 90 (8), 65 (12), 28 (7) | |

300 MHz-¹H-NMR (DMSO):

| | | |
|---|---|---|
| δ= | 8.17 | (m_{c}, 1H, Hetaryl arom.) |
| | 8.05-8.08 | (m_{c}, 2H, arom.) |
| | 7.99 | (m_{c}, 1H, Hetaryl arom.) |
| | 7.73-7.77 | (m_{c}, 2H, Hetaryl arom.) |
| | 7.56-7.57 | (m_{c}, 1H, arom.) |
| | 7.53 | (m_{c}, 1H, arom.) |
| | 7.42-7.48 | (m_{c}, 1H, arom.) |
| | 7.22 | (s, 1H, Hetaryl arom.) |

¹³C-NMR (DMSO):

| | | |
|---|---|---|
| δ= | 166.65 | (C=O) |
| | 150.94 | (C_{quart}, arom.) |
| | 142.61 | (C_{quart,} arom.) |
| | 140.74 | (C_{quart,} arom.) |
| | 139.59 | (C_{quart,} arom.) |
| | 138.49 | (C_{quart}, arom.) |
| | 131.13 | (C-H, arom.) |
| | 130.12 | (C-H, arom.) |
| | 129.81 | (C-H, arom.) |
| | 129.59 | (C-H, arom.) |
| | 129.01 | (C-H, arom.) |
| | 128.67 | (C-H, arom.) |
| | 128.44 | (C-H, arom.) |
| | 127.99 | (C-H, arom.) |
| | 127.27 | (C-H, arom.) |
| | 102.02 | (C-3, Pyrrol) |

### Charakterisierung von 2-(Triisopropyl)silyl-pyrrolo[2,3-b] chinoxalin (D)

| | | |
|---|---|---|
| Wellenzahl[cm-1] = | 3467cm⁻¹ | (ν (NH), NR₂) |
| | 3138 | (ν (C-H), Hetaryl) |
| | 2967 | (ν (C-H), Alkyl) |
| | 1637 | (γ (C=C), Hetaryl) |
| | 1558 | (γ (C=C), Hetaryl) |
| | 1440 | (γ (C=C), Hetaryl) |
| | 1387 | ((Gerüst), Hetaryl) |
| | 1249 | ((Gerüst), Hetaryl) |
| | 880 | (γ (Si-C), SiR₃) |
| | 762 | (γ (C-H), Hetaryl) |
| MS(70eV):*m*/*z* (%)= | 325 [M⁺] (42), 282 (100), 254 (20), 240 (18), 226 (16), 212 (24), 196 (5), 181 (1), 153 (3), 113 (11), 84 (16),74 (6), 43 (7) | |

300 MHz-¹H-NMR (CDCl₃):

| | | |
|---|---|---|
| δ= | 7.68-7.80 | (m_{c}, 1H, Hetaryl arom.) |
| | 7.51-7.56 | (m_{c}, 2H, Hetaryl arom.) |
| | 7.35-7.40 | (m_{c}, 1H, Hetaryl arom.) |
| | 7.09 | (s, 1H, Hetaryl arom.) |

### Charakterisierung von N-[4-(Acetyl)-phenyl]-pyrrolo[2,3-b] chinoxalin (E)

IR (KBr):

| | | |
|---|---|---|
| Wellenzahl [cm⁻¹] = | 3131 | (ν (NH), NR₂) |
| | 3052 | (ν (C-H), Ph) |
| | 1690 | (γ (C=O), COCH₃) |
| | 1611 | (γ (C=C), Ph) |
| | 1499 | (γ (C=C), Ph) |
| | 1420 | ((Gerüst), Ph) |
| | 1348 | ((Gerüst), Ph) |
| | 848 | (γ (Si-C), SiR₃) |
| | 782 | (ν (C-H), Ph) |
| MS(70eV):*m*/*z*(%)= | 364 [M⁺+1] (35), 363 [M⁺] (100), 362 [M⁺-1] (46), 348 (9), 320 (69), 294 (5), 174 (5), 127 (12), 84 (13), 70 (48), 43 (34) | |

300 MHz-¹H-NMR (CDCl₃):

| | | |
|---|---|---|
| δ= | 8.36 | (m_{c}, 1H, Hetaryl arom.) |
| | 8.04-8.08 | (m_{c}, 3H, Hetaryl arom.) |
| | 7.69-7.70 | (m_{c}, 2H, arom.) |
| | 7.50-7.52 | (m_{c}, 2H, arom.) |
| | 7.38-7.42 | (m_{c}, 5H, arom.) |
| | 6.82 | (s, 1H, Hetaryl arom.) |
| | 2.64 | (s, 3H, CH₃) |

300 MHz-¹³C-NMR (CDCl₃):

| | | |
|---|---|---|
| δ= | 197.03 | (C_{quart}, arom.) |
| | 151.41 | (C_{quart}, arom.) |
| | 144.40 | (C_{quart}, arom.) |
| | 141.72 | (C_{quart}, arom.) |
| | 140.08 | (C_{quart}, arom.) |
| | 139.50 | (C_{quart}, arom.) |
| | 135.87 | (C_{quart}, arom.) |
| | 130.69 | (C-H, arom.) |
| | 129.78 | (C-H, arom.) |
| | 129.22 | (C-H, arom.) |
| | 128.86 | (C-H, arom.) |
| | 128.76 | (C-H, arom.) |
| | 128.05 | (C-H, arom.) |
| | 128.02 | (C-H, arom.) |
| | 127.72 | (C-H, arom.) |
| | 127.36 | (C-H, arom.) |
| | 102.75 | (C-3, Pyrrol) |
| | 26.63 | (CH₃) |

### Beispiel C: Synthese von 1-H-Pyrrolo [2,3-b] chinoxalin, 2-Phenyl-1H-pyrrolo [2,3-b] chinoxalin und 2-(Triisopropyl)silyl-1-H-pyrrolo [2,3-b] chinoxalin

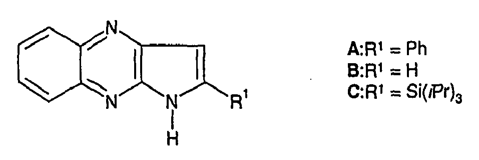

Herstellung: Zu einer Lösung von (1,5 mmol) 3-Alkinyl-2-amino-chinoxalin in 1,5 ml NMP (N-Methylpyrrolidon; wasserfrei) werden unter Inertgasatmosphäre KO*t*Bu (Kalium-*tert*-butoxid, 1.95 mmol) zugegeben und 4 - 6 h bei Raumtemparatur rühren lassen. Anschließend werden 1,5 ml H₂O zugesetzt und mit Dichlormethan (3x10 ml) extrahiert. Nach Waschen mit NaCl-Lösung und Trocknen über MgSO₄ wird der Rückstand durch Säulenchromatographie an Kieselgel mit Cyclohexan/Essigester (2:1) gereinigt. Die 1-*H*-Pyrrolo [2,3-*b*] chinoxaline lassen sich in Ausbeuten von 64-97 % gewinnen.

### Charakterisierung von 2-Phenyl-1H-pyrrolo [2,3-b] chinoxalin (A)

IR (KBr):

| | | |
|---|---|---|
| Wellenzahl [cm⁻¹] = | 3107 | (ν (N-H), NH) |
| | 3085 | (ν (C-H), Ph) |
| | 1604 | (ν (C=C), Ph) |
| | 1564 | (ν (C=C), Ph) |
| | 1499 | (ν (C-H), Ph) |
| | 1341 | ((Gerüst), Ph) |
| | 848 | (γ (C-H), Ph) |
| | 759 | (γ (C-H), Ph) |

300 MHz-¹H-NMR (DMSO):

| | | |
|---|---|---|
| δ= | 12.55 | (br, s, 1H, NH) |
| | 8.14-8.17 | (m, 2H, Hetaryl arom.) |
| | 8.07-8.10 | (m, 1H, Hetaryl arom.) |
| | 8.03-8.05 | (m, 1H, Hetaryl arom.) |
| | 7.68-7.72 | (m, 2H, arom.) |
| | 7.53-7.60 | (m, 3H, arom.) |
| | 7.30 | (s, 1H, Hetaryl arom.) |

75 MHz-¹³C-NMR (DMSO):

| | | |
|---|---|---|
| δ= | 149.30 | (C_{quart},arom.) |
| | 143.65 | (C_{quart}, arom.) |
| | 140.32 | (C_{quart}, arom.) |
| | 138.78 | (C_{quart}, arom.) |
| | 130.13 | (C-H, arom.) |
| | 129.14 | (C-H, arom.) |
| | 128,54 | (C-H, arom.) |
| | 127.83 | (C-H, arom.) |
| | 127.09 | (C-H, arom.) |
| | 126.43 | (C-H, arom.) |
| | 126.32 | (C-H, arom.) |
| | 95.56 | (C-3, Pyrrol) |
| MS(70eV):*m*/*z*(%)= | 246 (15) [M⁺+1], 245 (100) [M⁺], 244 (6), 142 (11), 128 (7), 116(8), 102 (24), 90 (22), 77 (21) | |

### Charakterisierung von 1-H-Pyrrolo [2,3-b] chinoxalin (B)

IR (KBr):

| | | |
|---|---|---|
| Wellenzahl [cm⁻¹]= | 3105 | (ν (N-H), NH) |
| | 2940 | (ν (C-H), Pyrrol) |
| | 1564 | (ν (C=C), Hetaryl) |
| | 1535 | (ν (C=C), Hetaryl) |
| | 1341 | ((Gerüst), Hetaryl) |
| | 762 | (γ (C-H), Hetaryl) |

300 MHz-¹H-NMR (CDCl₃):

| | | |
|---|---|---|
| δ= | 12.07 | (br.s, 1H, NH) |
| | 8.30-8.31 | (m, 1H, Hetaryl arom.) |
| | 8.12-8.15 | (m, 1H, Hetaryl arom.) |
| | 8.05-8.09 | (m, 1H, Hetaryl arom.) |
| | 7.68-7.75 | (m, 1H, Hetaryl arom.) |
| | 7.58-7.64 | (m, 1H, H-2) |
| | 6.74-6.76 | (m, 1H, H-3) |
| MS(70eV):*m*/*z* (%)= | 169 (100) [M⁺], 168 (6), 142 (14), 129 (1), 118 (7), 102(8), 90 (5), 76 (2) | |

### Charakterisierung von 2-(Triisopropyl)silyl-1-H-pyrrolo [2,3-b] chinoxalin (C)

IR (KBr):

| | | |
|---|---|---|
| Wellenzahl [cm⁻¹] = | 3302 | (ν (N-H), NH) |
| | 2966 . | (ν (C-H), i-R) |
| | 1637 | (ν (C=C), Hetaryl) |
| | 1611 | (ν (C=C), Hetaryl) |
| | 1558 | (ν (C=C), Hetaryl) |
| | 1505 | (ν (C=C), Hetaryl) |
| | 1249 | ((Gerüst), Hetaryl) |
| | 880 | (γ (Si-C), SiR₃) |
| | 762 | (γ (C-H), Hetaryl) |

300 MHz-¹H-NMR (CDCl₃):

| | | |
|---|---|---|
| δ= | 7.83-7.85 | (m, 1H, Hetaryl arom.) |
| | 7.50-7.53 | (m, 2H, Hetaryl arom.) |
| | 7.37-7.39 | (m, 1H, Hetaryl arom.) |
| | 7.34 | (s, 1H, Hetaryl arom.) |
| | 5.51 | (br.s, 1H, NH) |
| | 1.15 | (sep, 3H, CH) |
| | 1.13 | (s, 9H, CH₃) |
| | 1.09 | (s, 9H, CH₃) |
| MS(70eV):*m*/*z* (%)= | 325 [M⁺] (36), 298 (9), 282 (100), 266 (1), 254 (31), 240 (8), 226 (14), 212 (20), 196 (5), 170 (1), 153 (9), 112 (14), 98 (3), 84 (26), 76 (8) | |

### Beispiel D: Synthese von

A: 1,4-Dihydro-pyrazino [2,3-b] chinoxalin-2,3-dion
B: 1 Hydro-pyrazino [2,3-b] chinoxalin-2,3-on

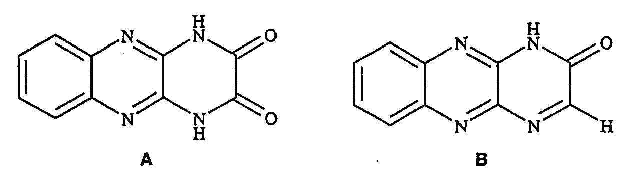

Herstellung: Glyoxalsäure-Mono-hydrat bzw. Oxalsäure-Dihydrat (1.10 mmol) wird zu einer Lösung von 2,3-Diaminochinoxalin (1,0 mmol) in Ethanol (wasserfrei) gegeben, und unter Zusatz einiger Tropfen Eisessig 48 h bei Raumtemparatur rühren lassen. Nach Einengen der Lösung scheidet sich ein Niederschlag ab, der durch Waschen mit kaltem Ethanol (A) bzw. durch Säulenchromatographie (B) an Kieselgel mit einem Eluentengemisch Essigester/Ethanol gereinigt werden kann. Die kondensierten Chinoxaline sind in Form blaßgelber Pulver in einer Ausbeute von 68 (A) und 79% (B) erhältlich.

### Charakterisierung von 1,4-Dihydro-pyrazino [2,3-b] chinoxalin-2,3-(A)

IR (KBr):

| | | |
|---|---|---|
| Wellenzahl [cm⁻¹] = | 3309 | (ν (N-H), NH) |
| | 3052 | (ν (C-H), Hetaryl) |
| | 1690 | (ν (C=O)) |
| | 1624 | (ν (C=C), Hetaryl) |
| | 1506 | (ν (C=C), Hetaryl) |
| | 1249 | ((Gerüst), Hetaryl) |
| | 755 | (γ (C-H), Hetaryl) |

300 MHz-¹H-NMR (CDCl₃):

| | | |
|---|---|---|
| δ= | 11.96 | (br.s, 2H, NH) |
| | 7.16-7.21 | (m, 2H, Hetaryl arom.) |
| | 7.09-7.14 | (m, 2H, Hetaryl arom.) |

75 MHz-¹³C-NMR (CDCl₃):

| | | |
|---|---|---|
| δ= | 154.43 | (C_{quart}, C=O) |
| | 126.27 | (C_{quart} arom.) |
| | 124.54 | (C_{quart} arom.) |
| | 124.78 | (C-H, arom.) |
| | 123.12 | (C-H, arom.) |
| | 122.17 | (C-H, arom.) |
| | 114.31 | (C-H, arom.) |
| MS(70eV):*m*/*z* (%)= | 214 (2) [M⁺], 186 (1), 160 (100), 144 (10), 133 (88), 116(4), 106 (51), 90 (25), 79 (26) | |

### Charakterisierung von 1-Hydro-pyrazino [2,3-b] chinoxalin-2,3-(B)

IR (KBr):

| | | |
|---|---|---|
| Wellenzahl [cm⁻¹] = | 3374 | (ν (N-H), NH) |
| | 3058 | (ν (C-H), Hetaryl) |
| | 1703 | (ν (C=O)) |
| | 1644 | (ν (C=N), Hetaryl) |
| | 1585 | (ν (C=C), Hetaryl) |
| | 1493 | ((Gerüst), Hetaryl) |
| | 1341 | ((Gerüst), Hetaryl) |
| | 1262 | ((Gerüst), Hetaryl) |
| | 762 | (γ (C-H), Hetaryl) |

300 MHz-¹H-NMR (CDCl₃):

| | | |
|---|---|---|
| δ= | 11.93 | (br.s, 1H, NH) |
| | 9.21 | (s, 1H, H-3) |
| | 7.61-7.73 | (m_{c}, 2H, H-5, H-8) |
| | 7.43-7.56 | (m_{c}, 2H, H-6, H-7) |
| MS(70eV):*m*/*z* (%)= | 198 (96) [M⁺], 186 (16), 170 (76), 158 (7), 143 (100), 133 (5), 116(52), 102 (19), 90 (34), 76 (14), 63 (12) | |

### Beispiel E: Synthese der 2-Aryl-1H-imidazo [4,5-b]chinoxaline bzw. der 2-Aryl-1-methyl-imidazo [4,5-b]chinoxaline

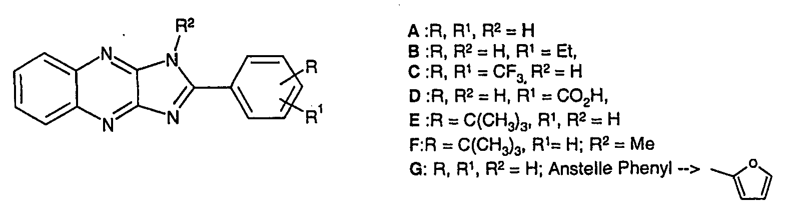

Herstellung: Zu einer Lösung von 1.00 mmol Diaminochinoxalin (160 mg) in 15.0 ml eines polaren aprotischen Lösungsmittels (vorzugsweise THF oder Dioxan/ getrocknet) werden 0.98 mmol des Aroylchlorids (frisch destilliert) gegeben und unter Rückfluß 48-72 h erhitzt. Nach dem Abkühlen wird das Lösungsmittel entfernt und der Rückstand säulenchromatographisch an Kieselgel mit einem Lösungsmittel-Eluenten-Gemisch Cyclohexan/Essigester → Essigester/Ethanol aufgereinigt. Die Produkte können als hellgelbe Pulver in einer Ausbeute von 33 - 76 % gewonnen werden.
A: 2-Phenyl-1*H*-imidazo [4,5-*b*]chinoxalin
B: 2-[4-[(Ethyl)phenyl]-1*H*-imidazo [4,5-*b*]chinoxalin
C: 2-[3,5-Bis(trifluormethyl)phenyl]-1*H*-imidazo [4,5-*b*]chinoxalin
D: 2-[4-(Carboxy)phenyl]-1*H*-imidazo [4,5-*b*]chinoxalin
E: 2-[4-[(*t*-Butyl) phenyl]-1*H*-imidazo [4,5-*b*]chinoxalin
F: 2-[4-[(*t*-Butyl) phenyl]-1-methyl-imidazo [4,5-*b*]chinoxalin
G: 2-(2 Furyl)-1*H*-imidazo [4,5-*b*]chinoxalin

### Charakterisierung von 2-Phenyl-1H-imidazo [4,5-b]chinoxalin (A)

IR (KBr):

| | | |
|---|---|---|
| Wellenzahl [cm⁻¹] = | 3058 | (ν (C-H), Ph) |
| | 1611 | (ν (C=C), Ph) |
| | 1538 | (ν (C=C), Ph) |
| | 1348 | ((Gerüst), Ph) |
| | 755 | (γ (C-H), Ph) |

300 MHz-¹H-NMR (CDCl₃):

| | | |
|---|---|---|
| δ= | 12.86 | (br. s, 1H, NH) |
| | 8.42-8.45 | (m, 2H, Hetaryl arom.) |
| | 8.11-8.16 | (m, 2H, Hetaryl arom.) |
| | 7.75-7.80 | (m, 2H, arom.) |
| | 7.66-7.72 | (m, 3H, arom.) |
| MS(70eV):*m*/*z*(%)= | 247 (16.2) [M⁺+1], 246 (100) [M⁺], 245 (4), 219 (5), 143 (52), 116(24), 104 (4), 90 (6), 77 (4) | |

### Charakterisierung von2-[4-[(Ethyl) phenyl]-1H-imidazo [4,5-b]chinoxalin (B)

IR(KBr):

| | | |
|---|---|---|
| Wellenzahl [cm⁻¹] = | 3039 | (ν (C-H), Ph) |
| | 2960 | (ν (C-H), C₂H₅) |
| | 1617 | (ν (C=C), Ph) |
| | 1545 | (ν (C=C), Ph) |
| | 1492 | (ν (C=C), Ph) |
| | 1335 | ((Gerüst), Ph) |
| | 769 | (γ (C-H), Ph) |

300 MHz-¹H-NMR (CDCl₃):

| | | |
|---|---|---|
| δ= | 13.84 | (br. s, 1H, NH) |
| | 8.32-8.35 | (m, 2H, arom.) |
| | 8.12 | (m, 2H, Hetaryl arom.) |
| | 7.74-7.78 | (m, 2H, Hetaryl arom.) |
| | 7.52 | (m, 2H, arom.) |
| | 2.74 | (q, 2H, CH₂) |
| | 1.25 | (t, 3H, CH₃ |

75 MHz-¹³C-NMR (CDCl₃):

| | | |
|---|---|---|
| δ= | 167.23 | (C_{quart.}, arom.) |
| | 166.27 | (C_{quart.}, arom.) |
| | 161.97 | (C_{quart.}, arom.) |
| | 155.12 | (C_{quart.}, arom.) |
| | 149.03 | (C_{quart.}, arom.) |
| | 147.18 | (C_{quart.}, arom.) |
| | 131.63 | (C_{quart.}, arom.) |
| | 130.15 | (C-H, arom.) |
| | 129.35 | (C-H, arom.) |
| | 128.64 | (C-H, arom.) |
| | 128.15 | (C-H, arom.) |
| | 127.99 | (C-H, arom.) |
| | 127.88 | (C-H, arom.) |
| | 127.73 | (C-H, arom.) |
| | 127.50 | (C-H, arom.) |
| | 28.17 | (CH₂CH₃) |
| | 15.29 | (CH₂CH₃) |
| MS(70eV):*m*/*z* (%)= | 275 (19) [M⁺+1], 274 (100) [M⁺], 273 (14), 245 (3), 259 (39, 143 (11), 116 (14), 102 (2), 89 (3), 77 (1) | |

(HRMS) (hochaufgelöste Masse) C₁₇H₁₄N₄ [M⁺]
Ber. für:274, 1219
Gef. für:274,1217

### Charakterisierung von 2-[3,5-Bis(trifluormethyl)phenyl]-1H-imidazo [4,5-b] chinoxalin (C)

IR (KBr):

| | | |
|---|---|---|
| Wellenzahl [cm⁻¹] = | 3065 | (ν (C-H), Ph) |
| | 1657 | (ν (C=C), Ph) |
| | 1282 | (ν (C=F), Ph) |
| | 1181 | ((Gerüst), Ph) |
| | 781 | (γ (C-H), Ph) |

300 MHz-¹H-NMR (CDCl₃):

| | | |
|---|---|---|
| δ= | 11.83 | (br. s, 1H, NH) |
| | 9.94 | (m, 2H, arom.) |
| | 8.25 | (m, 1H, arom.) |
| | 8.00-8.06 | (m, 2H, Hetaryl arom.) |
| | 7.65-7.70 | (m, 2H, Hetaryl arom.) |
| MS(70eV):*m*/*z* (%)= | 383 (20) [M⁺+1], 382 (100) [M⁺], 381 (3), 363 (9), 313 (1), 220 (2), 170 (2), 143 (90, 116 (39), 102 (5), 90 (19), 76 (2) | |

### Charakterisierung von 2-[4-(Carboxy)phenyl]-1H-imidazo [4,5-b]chinoxalin (D)

IR (KBr):

| | | |
|---|---|---|
| Wellenzahl [cm⁻¹] = | 3472 | (ν (O-H), OH) |
| | 3056 | (ν (C-H), Ph) |
| | 1697 | (ν (C=O), CO₂H) |
| | 1637 | (ν (C=C), Ph), |
| | 1512 | (ν (C=C), Ph) |
| | 1472 | (ν (C=C), Ph) |
| | 1341 | ((Gerüst), Ph) |
| | 1288 | ((Gerüst), Ph) |
| | 755 | (γ (C-H), Ph) |

300 MHz-^{1H}-NMR (CDCl₃):

| | | |
|---|---|---|
| δ= | 11.93 | (br. s, 1H, NH) |
| | 8.49-8.52 | (m, 1H, arom.) |
| | 8.13-8.14 | (m, 1H, arom.) |
| | 8.10-8.12 | (m, 2H, Hetaryl arom.) |
| | 7.71-7.75 | (m, 2H, Hetaryl arom.) |
| | 7.30-7.34 | (m, 1H, arom.) |
| | 7.12-7.10 | (m, 1H, arom.) |
| | 6.75 | (br. s, 1H, OH) |
| MS(70eV):*m*/*z* (%)= | 290 [M⁺] (5), 169 (7), 160 (97), 149 (16), 133 (100), 116 (8), 105 (13), 91 (34), 79 (23) | |

### Charakterisierung von 2-(2-Furyl)-1H-imidazo [4,5-b]chinoxalin (G)

IR (KBr):

| | | |
|---|---|---|
| Wellenzahl [cm⁻¹] = | 3111 | (ν (C=H), Furyl) |
| | 2960 | (ν (C-O-C), Furyl) |
| | 1623 | (ν (C=C), Hetaryl) |
| | 1525 | (ν (C=C), Hetaryl) |
| | 1327 | ((Gerüst), Hetaryl) |
| | 1268 | ((Gerüst), Hetaryl) |
| | 775 | (γ (C-H), Hetaryl) |

300 MHz-¹H-NMR (CDCl₃):

| | | |
|---|---|---|
| δ= | 14.06 | (br. s, 1H, NH) |
| | 8.26-8.27 | (m, 1H, Furyl arom.) |
| | 8.14-8-19 | (m, 2H, Hetaryl arom.) |
| | 7.98-8.04 | (m, 2H, Hetaryl arom.) |
| | 7.78-7.74 | (m, 1H, Furyl arom.) |
| | 6.96-6.98 | (m, 1H, Furyl arom.) |
| MS(70eV):*m*/*z* (%)= | 238 (13) [M⁺+1], 237 (100 [M⁺], 236 (3), 208 (2), 161 (25), 143 (62), 133 (11), 116 (32), 105 (9), 95 (12), 90 (7), 77 (5) | |

### Charakterisierung von 2-[4-[(t-Butyl) phenyl]-1-methyl-imidazo [4,5-b]chinoxalin (F)

(HRMS) (hochaufgelöste Masse) (El) C₂₀H₂₀N₄ [M⁺]
Ber. für: 316.1697
Gef. für: 316.1701
IR (KBr):

| | | |
|---|---|---|
| Wellenzahl [cm⁻¹] = | 3065 | (ν (C-H), Ph) |
| | 2960 | (ν (C-H), Alkyl) |
| | 1617 | (γ (C=C), Ph) |
| | 1545 | (γ (C=C), Ph) |
| | 1485 | (γ (C=C), Ph) |
| | 1446 | (γ (C=C), Ph) |
| | 1382 | ((Gerüst), Ph) |
| | 1341 | ((Gerüst), Ph) |
| | 1123 | ((Gerüst), Ph) |
| | 867 | (γ (C-H), Ph) |
| | 755 | (γ (C-H), Ph) |
| MS(70eV):*m*/*z* (%)= | 317 [M⁺ +1], 316 [M⁺] (65), 301 (100), 273 (7), 258 (5), 150(12), 128 (21), 103 (9), 90 (6), 77 (3), 57 (1) | |

300 MHz-¹H-NMR (CDCl₃):

| | | |
|---|---|---|
| δ= | 8.17-8.20 | (m_{c}, 1H, H-8', Hetaryl arom.) |
| | 8.03-8.07 | (m_{c}, 1H, H-7', Hetaryl arom.) |
| | 7.88-7.91 | (m_{c}, 2H, H-2, H-6, arom.) |
| | 7.61-7.64 | (m_{c}, 1H, H-6', H-9' Hetaryl arom.) |
| | 7.52-7.55 | (m_{c}, 2H, H-3, H-5, arom. |
| | 4.09 | (s, 3H, CH₃) |
| | 1.30 | (s, 9H, C(CH₃)₃) |

¹³C-NMR (CDCl₃);

| | | |
|---|---|---|
| δ= | 163.49 | (C-2') |
| | 155.44 | (C-4) |
| | 150.17 | (C-5') |
| | 143.43 | (C-4') |
| | 141.79 | (C-10') |
| | 139.95 | (C-9') |
| | 129.68 | (C-H) |
| | 128.13 | (C-H) |
| | 128.05 | (C-H) |
| | 127.43 | (C-H) |
| | 126.13 | (C-3) |
| | 125.72 | (C-5) |
| | 35.24 | (C(CH₃)₃) |
| | 31.24 | (C(CH₃)₃) |
| | 29.85 | (CH₃) |

### Beispiel F: Synthese von 2-Aryl-4-methyl-imidazo [4,5-b]chinoxalinen

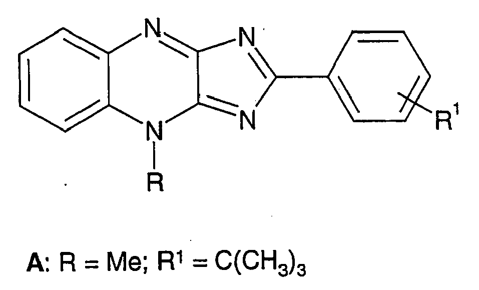

### Charakterisierung von 2-[4-[(t-Butyl) phenyl]-4-methyl-imidazo [4,5-b]chinoxalin (A)

IR (KBr):

| | | |
|---|---|---|
| Wellenzahl [cm⁻¹] = | 3058 | (ν (C-H), Ph) |
| | 2954 | (ν (C-H), Alkyl) |
| | 2861 | (ν (C-H), Alkyl) |
| | 1604 | (γ (C=C), Ph) |
| | 1486 | (γ (C=C), Ph) |
| | 1454 | (γ (C=C), Ph) |
| | 1354 | ((Gerüst), Ph) |
| | 1259 | ((Gerüst), Ph) |
| | 769 | (γ (C-H), Ph) |
| MS(70eV):*m*/*z* (%)= | 317 [M⁺+1] (32), 302 (100), 273 (3), 158 (1), 131 (2) | |

300 MHz-¹H-NMR (CDCl₃):

| | | |
|---|---|---|
| δ= | 8.51-8.54 | (m_{c}, 2H, H-2, H-6, arom.) |
| | 8.23-8.26 | (m_{c}, 1H, H-8', Hetaryl arom.) |
| | 7.74-7.78 | (m_{c}, 1H, H-7', Hetaryl arom.) |
| | 7.67-7.70 | (m_{c}, 1H, H-6', Hetaryl arom.) |
| | 7.59-7.65 | (m_{c}, 1H, H-9', Hetaryl arom.) |
| | 7.48-7.51 | (m_{c}, 2H, H-3, H-5, arom.) |
| | 4.43 | (s, 3H, CH₃) |
| | 1.28 | (s, 9H, C(CH₃)₃) |

¹³C-NMR (CDCl₃):

| | | |
|---|---|---|
| δ= | 163.51 | (C-2') |
| | 156.35 | (C-4) |
| | 138.21 | (C-5') |
| | 131.26 | (C-4') |
| | 130.50 | (C-H) |
| | 129.79 | (C-H) |
| | 128.92 | (C-H) |
| | 125.83 | (C-H) |
| | 114.73 | (C-5') |
| | 35.24 | ((CH₃)₃) |
| | 34.28 | (CH₃) |
| | 31.26 | (C(CH₃)₃) |

### Beispiel G: Synthese von 2-tert-Butyl-1H-imidazo-chinoxalin

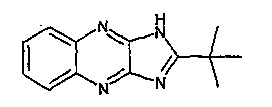

Herstellung: Zu einer Lösung von 1,00 mmol Diaminochinoxalin (160 mg) in 15,0 ml eines polaren aprotischen Lösungsmittels (vorzugsweise THF oder Dioxan/ getrocknet) werden 0.98 mmol des Pivaloylanhydrids) (206 mg) gegeben und unter Rückfluß 48-72h erhitzt. Nach dem Abkühlen wird das Lösungsmittel am Rotationsverdampfer entfernt und der Rückstand säulenchromatographisch an Kieselgel mit einem Lösungsmittel-Eluenten Cyclohexan/Essigester → Essigester/Ethanol aufgereinigt. Das 2-*tert*-Butyl-1*H-*imidazo-chinoxalin kann als beige- weißes Pulver erhalten werden.
IR (KBr):

| | | |
|---|---|---|
| Wellenzahl [cm⁻¹] | 3355 | (ν(N-H), NH) |
| | 3085 | (ν(C-H), Ph) |
| | 2980 | (ν(C-H), CH₃) |
| | 1623 | (ν(C=N),Hetaryl) |
| | 1611 | (ν(C=C), Ph) |
| | 1518 | (ν(C=C), Ph) |
| | 1334 | ((Gerüst), Ph) |
| | 768 | (γ(C-H), Aryl) |

¹H-NMR (300 MHz, d-CDCl₃):

| | | |
|---|---|---|
| δ= | 11.13 | (br.s, 1H, NH) |
| | 8.16-8.18 | (m, 2H, Hetaryl, arom.) |
| | 7.71-7.74 | (m, 2H, Hetaryl, arom.) |
| | 1.60 | (s, 9H, CH₃) |

¹³C (75,4 MHz)

| | | |
|---|---|---|
| δ = | 158.27;140.44; | (C_{quart}, Hetaryl, arom.) |
| | 128.40; 125.18 | |
| | 128.80; 127,89 | (C-H, Hetaryl, arom.) |
| | | |
| | 34.92 | (C_{quart}, C(CH₃)₃)) |
| | 28.88 | (C(CH₃)) |

MS:

| | |
|---|---|
| *m*/*z (%)* = | 226 (32) [M⁺], 211 (100), 184 (17), 161 (5), 144 (14), 133 (3), 116 (14), 102 (3), 90 (10), 71 (18) |

### Beispiel H: Herstellung von 1-(2-Ethyl-hexyl)-2-phenyl-imidazo[3,4-b]chinoxalin

### a) Route A: Herstellung von 2-Amino-3-chlor-chinoxalin

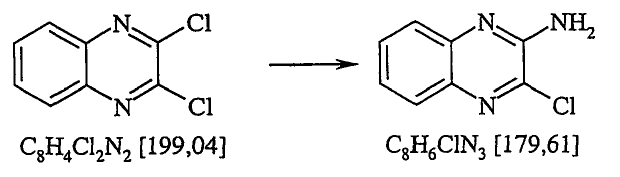

2,1 g (10,4 mmol) 2,3-Dichlor-chinoxalin werden mit 50 ml 20%igem ethanolischen Ammoniak in einem Autoklaven bei 80° - 85°C und Eigendruck für 8 h gerührt. Die Reaktionslösung wird zum Rückstand einrotiert und das Rohprodukt über Kieselgel mit Toluol/Ethylacetat chromatographiert. Die produkthaltigen Fraktionen werden gesammelt und einrotiert. Ausbeute: 1,18 g (63 %)

### b) Route B: Herstellung von 2-Chlor-3-(2-ethyl-hexylamino-)-chinoxalin

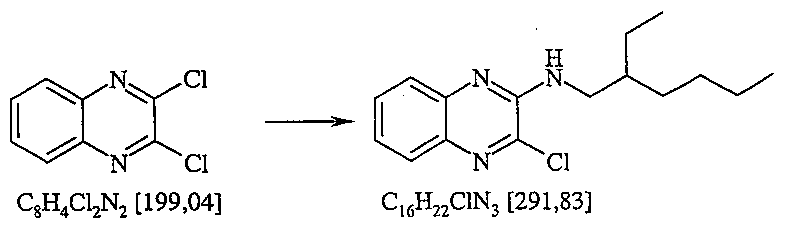

12 g (60,3 mmol) 2,3-Dichlor-chinoxalin werden mit 9,5 g (72,1 mmol) Diisopropylethylamin in 125 ml THF gelöst. Zu der Lösung werden 8,5 g (66 mmol) 2-Ethyl-hexylamin zugetropft und die Reaktion für insgesamt 48 h zum Rückfluss erhitzt bis nach DC (Kieselgel: n-Heptan/MTB-Ether) kein Edukt mehr sichtbar ist. Die Reaktionslösung wird zum Rückstand einrotiert und das Rohprodukt über Kieselgel mit n-Heptan/MTBEther chromatographiert. Die produkthaltigen Fraktionen werden gesammelt und einrotiert. Ausbeute: 11,8 g (67 %)

### c) Route A: Herstellung von 2-Amino-3-(2-ethyl-hexylamino-)-chinoxalin

### aus 2-Amino-3-chlor-chinoxalin

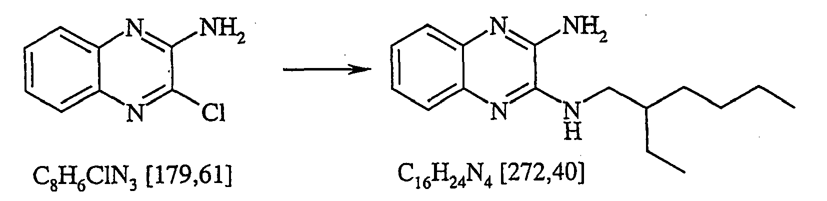

1,8 g (10 mmol) 2-Amino-3-chlor-chinoxalin werden mit 2,5 g (19 mmol) Diisopropylethylamin in 85 ml THF gelöst. Zu der Lösung werden 1,6 g (12 mmol) 2-Ethyl-hexylamin zugetropft und die Reaktion für insgesamt 48 h zum Rückfluss erhitzt bis nach DC (Kieselgel: n-Heptan/MTB-Ether) kein Edukt mehr sichtbar ist. Die Reaktionslösung wird zum Rückstand einrotiert und das Rohprodukt über Kieselgel mit Toluol/Ethylacetat chromatographiert. Die produkthaltigen Fraktionen werden gesammelt und einrotiert. Ausbeute: 1,9 g (71 %)

### d) Route B: Herstellung von 2-Amino-3-(2-ethyl-hexylamino-)-chinoxalin

### aus 2-Chlor-3-(2-ethylhexylamino-)-chinoxalin

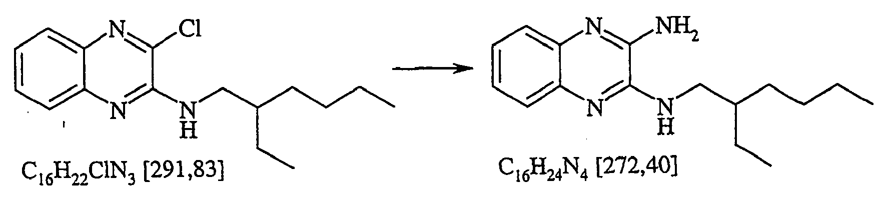

11,5 g 2-Chlor-3-(2-ethylhexylamino-)-chinoxalin werden mit 200 ml 20%igem ethanolischen Ammoniak bei 90° - 100°C und Eigendruck für 48 h gerührt. Nach dem Abkühlen des Autoklaven wird das Methanol abdestilliert und das Rohprodukt vom Edukt (10 %) durch Chromatographie über Kieselgel mit Toluol/Ethylacetat abgetrennt. Ausbeute: 7,9 g (73 %)

### e) Herstellung von 1-(2-Ethyl-hexyl)-2-phenyl-imidazo[3,4-b]chinoxalin

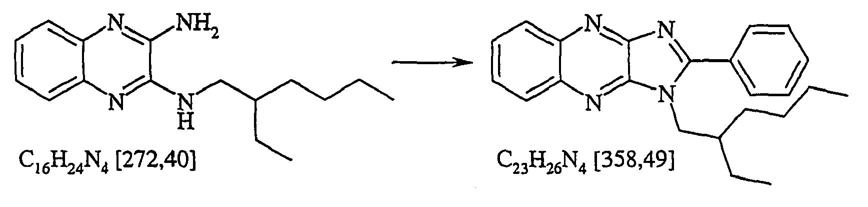

1,38 g (5,1 mmol) 2-Amino-3-(2-ethylhexylamino-)-chinoxalin werden in 20 ml DMF gelöst und die Lösung mit 0,94 g (5 mmol) Natriumdisulfit versetzt. Zu der Suspension werden 0,57 g (5,3 mmol) Benzaldehyd gegeben und die Reaktion bis zur vollständigen Umsetzung für 8 h auf 110°C erhitzt. Nach dem Abkühlen der Reaktionslösung auf Raumtemperatur werden 50 ml MTB-Ether und 50 ml Wasser zugegeben, die Phasen getrennt, die wässrige Phase mit 30 ml MTB-Ether nachextrahiert und die organischen Phasen zum Rückstand (1,73 g; 96 % Ausbeute) einrotiert. Das Rohprodukt wird zur weiteren Aufreinigung aus n-Heptan umkristallisiert. Ausbeute: 0,98 g (54 %)

Die für die einzelnen Umsetzungen a) - e) vorliegenden Spektren (MS und ¹H-NMR) entsprechen jeweils den Erwartungen.

### Beispiel J:

In der nachfolgenden Tabelle sind die Strukturformeln erfindungsgemäß einsetzbarer Chinoxalinderivate sowie die Maxima deren UV-A- bzw. UV-B-Absorption angegeben. Die Messung erfolgte in 2-Propanol bei einer Konzentration von 1 mg Substanz pro 100mL Lösungsmittel.

| **Strukturformel** | **UV-A** | | **UV-B** | |
|---|---|---|---|---|
| | **(400 320 nm)** | | **(280 320 nm)** | |
| | Max. Abs. | λ [nm] | Max. Abs. | λ [nm] |
| 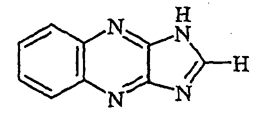 | 0,5227 | 328,0 | | |
| 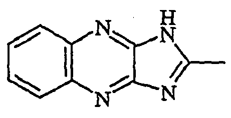 | 0,5824 | 327,5 | | |
| 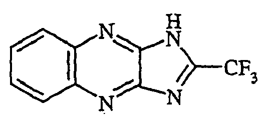 | 0,4875 | 327,5. | | |
| 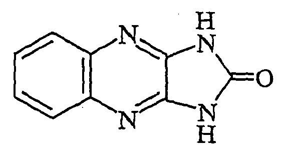 | 0,3754 | 342,0 | | |
| | 0,3381 | 335,0 | | |
| | 0,3707 | 327,5 | | |
| 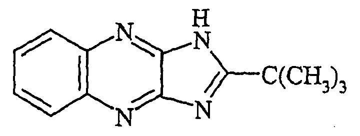 | 0,6290 | 330,0 | | |
| 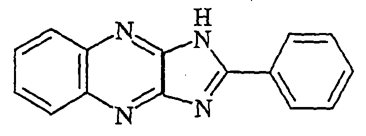 | 0,9293 | 368,0 | | |
| | 0,9565 | 354,0 | | |
| 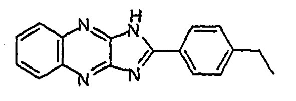 | 1,478 | 372,0 | | |
| | 1,4581 | 358,0 | | |
| | 0,7467 | 372,0 | | |
| | 0,7403 | 358,0 | | |
| 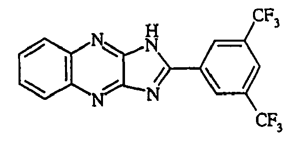 | 0,7037 | 358,0 | | |
| 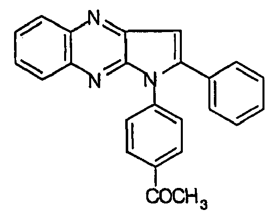 | 0,4067 | 359,0 | | |
| 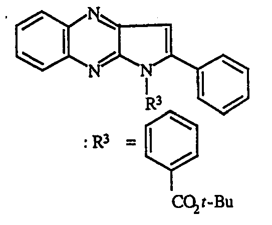 | 0,4496 | 358,0 | 0,5643 | 269,0 |
| 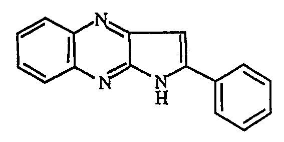 | 0,7158 | 367,0 | 0,8710 | 268,0 |
| 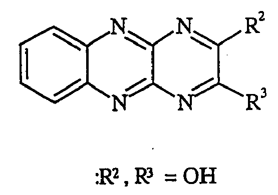 | 0,6909 | 327,0 | | |
| 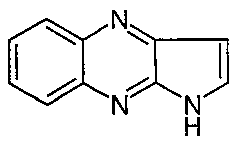 | 0,5381 | 338,0 | | |
| 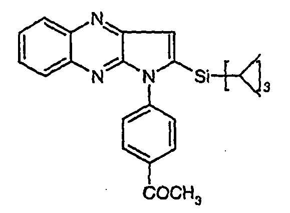 | 0,5774 | 403,0 | 0,4246 | 318,0 |
| 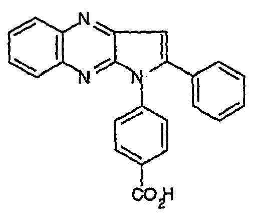 | 0,3685 | 358,0 | | |

### Anwendungsbeispiele

### Beispiel 1

**Herstellung eines erfindungsgemäßen Sonnenschutzsprays (O/W)**

| A | | % |
|---|---|---|
| 2-*tert*-Butyl-1*H*-imidazo-chinoxalin | (1) | 1,00 |
| Eusolex 2292 (Art.-Nr. 105382) | (1) | 7,50 |
| (Octyl Methoxycinnamate) | | |
| Eusolex HMS (Art.-Nr. 111412) | (1) | 7,00 |
| (Homosalate) | | |
| Volpo S-2 (Steareth-2) | (2) | 0,40 |
| Volpo S-10 (Steareth-10) | (2) | 0,80 |
| Pemulen TR-2 | (3) | 0,18 |
| (Acrylate/C10-39Alkyl Acrylate Crosspolymer) | | |
| Hetester PHA | (4) | 5,00 |
| (Propylene Glycol Isoceteth-3 Acetate) | | |
| Performa V 825 | (5) | 0,80 |
| (Synthetic Wax) | | |
| Dow Corning 200 (100cs) | (6) | 1,00 |
| (Dimethicone) | | |
| Oxynex K flüssig (Art.-Nr. 108324) | (1) | 0,10 |
| (PEG-8 (and) Tocopherol (and) Ascorbyl Palmitate (and) | | |
| Ascorbic Acid (and) Citric Acid) | | |

| B | | % |
|---|---|---|
| Eusolex 232 (Art.-Nr. 105372) | (1) | 1,00 |
| (Phenylbenzimidazole Sulfonic Acid) | | |
| Triethanolamin (Art.-Nr. 108379) | (1) | 0,90 |
| Propandiol-1,2 (Art.-Nr. 107478) | (1) | 2,00 |
| Konservierungsmittel | | q.s. |
| Wasser, demineralisiert | ad | 100,00 |

### Herstellung:

Zur Neutralisierung von Eusolex 232 wird das Triethanolamin ins Wasser der Phase B gegeben und Eusolex 232 unter Rühren zugegeben. Nach vollständiger Lösung werden die restlichen Rohstoffe der Phase B zugegeben und auf 80°C erhitzt. Phase A bis auf das Pemulen zusammengegeben und auf 80 °C erhitzen. Pemulen in Phase A einrühren. Phase B unter Rühren langsam zu Phase A geben, homogenisieren und unter Rühren abkühlen.

### Bemerkungen:

Als Konservierungsmittel werden verwendet:
0,05 % Propyl-4-hydroxybenzoat (Art. Nr. 107427)
0,15 % Methyl-4-hydroxybenzoat (Art. Nr. 106757)

### Bezugsquellen:

(1) Merck KGaA, Darmstadt
(2) Croda, Nettetal
(3) Goodrich, Neuss
(4) ROVI, Schlüchtern
(5) New Phase, NJ 08554
(6) Dow Corning, Wiesbaden

### Beispiel 2

**Herstellung eines erfindungsgemäßen Sonnenschutzgels (O/W)**

| A | | % |
|---|---|---|
| 2-*tert*-Butyl-1*H*-imidazo-chinoxalin | (1) | 1,00 |
| Eusolex 6300 (Art.-Nr. 5385) | (1) | 10,00 |
| Luvitol EHO | (2) | 2,00 |
| Dow Corning 200 (100 cs) | (3) | 2,00 |
| Sheabutter | (4) | 5,00 |
| Antaron V-220 | (5) | 2,00 |
| Oxynex K flüssig (Art.-Nr. 8324) | (6) | 1,00 |
| | | |

| B | | % |
|---|---|---|
| Eusolex 232 (Art.-Nr. 5372) | (1) | 0,75 |
| Tris(hydroxymethyl)-aminomethan (Art.-Nr. 8386) | (1) | 0,33 |
| Konservierungsmittel | | q.s. |
| Wasser, demineralisiert | | 20,00 |
| | | |

| C | | % |
|---|---|---|
| Tris(hydroxymethyl)-aminomethan (Art.-Nr. 8386) | (1) | 1,20 |
| Wasser, dem. | | 10,00 |
| | | |

| D | | % |
|---|---|---|
| Pemulen TR-1 | (6) | 0,60 |
| Wasser, dem. | ad | 100,00 |

### Herstellung:

Für Phase D das Pemulen TR-1 im Wasser homogen dispergieren und unter Rühren die vorgelöste Phase C zugeben. Zur Neutralisierung von Eusolex 232 wird das Tris(hydroxymethyl)-aminomethan im Wasser der Phase B gelöst und das Eusolex 232 unter Rühren zugegeben. Nach vollständiger Lösung werden die restlichen Rohstoffe der Phase B zugegeben. Phase B in Phase C/D einrühren. Phase A unter Erwärmen lösen und in Phase B/C/D einrühren. Homogenisieren.

### Bemerkungen:

Als Konservierungsmittel werden eingesetzt:
0,05 % Propyl-4-hydroxybenzoat (Merck-Art.-Nr. 7427)
0,15 % Methyl-4-hydroxybenzoat (Merck-Art.-Nr. 6757)

### Bezugsquellen:

(1) Merck KGaA, Darmstadt
(2) BASF, Ludwigshafen
(3) Dow Corning, Düsseldorf
(4) H. Erhard Wagner, Bremen
(5) GAF, Frechen
(6) Goodrich, Neuss

### Beispiel 3

**Herstellung einer Sonnenschutzlotion (W/O) mit UV-A/B-Schutz**

| A | | % |
|---|---|---|
| 2-[4[(Ethyl) phenyl]-1*H*-imidazo [4,5-*b*]chinoxalin | (1) | 1,00 |
| Eusolex 2292 (Art.-Nr. 1.05382) | (1) | 3,00 |
| Eusolex 4360 (Art.-Nr. 1.05376) | (1) | 2,00 |
| (Benzophenone-3) | | |
| Dehymuls E | (2) | 6,00 |
| (Dicocoyl Pentyerythrityl Citrate (and) Sorbitan Ses- | | |
| quioleate (and) Cera Alba (and) Aluminium Stearate) | | |
| Arlacel 989 | (3) | 1,00 |
| (PEG-7 Hydrogenated Castor Oil) | | |
| Bienenwachs (Art.-Nr. 1.11544) | (2) | 2,00 |
| Cetiol J 600 | (2) | 6,00 |
| (Oleyl Erucate) | | |
| Cetiol V | (2) | 6,00 |
| (Decyl Oleate) | | |
| Cetiol OE | (2) | 5,00 |
| (Dicaprylyl Ether) | | |
| Dow Corning 200 (100 cs) | (4) | 1,00 |
| (Dimethicone) | | |

| B | | % |
|---|---|---|
| Glycerin (etwa 87%) (Art.-Nr. 1.04091) | (1) | 5,00 |
| Magnesiumsulfat Heptahydrat (Art.-Nr. 1.05882) | (1) | 1,00 |
| Konservierungsmittel | | q.s. |
| Wasser, dem. | ad | 100,00 |

### Herstellung:

Phase B auf 80°C und Phase A auf 75 °C erhitzen. Phase B langsam in Phase A einrühren. Homogenisieren und unter Rühren abkühlen.

### Bemerkungen:

Als Konservierungsmittel sind enthalten:
0,05 % Propyl-4-hydroxybenzoat (Art. Nr. 1.07427)
0,15 % Methyl-4-hydroxybenzoat (Art. Nr. 1.06757)

### Bezugsquellen:

(1) Merck KGaA, Darmstadt
(2) Henkel KGaA, Düsseldorf
(3) ICl, Essen
(4) Dow Corning, Düsseldorf

### Beispiel 4

**Herstellung einer Sonnenschutzlotion mit IR3535^{™}**

| A | | | | % |
|---|---|---|---|---|
| 2-Phenyl-1*H*-imidazo [4,5-*b*]chinoxalin | | | (1) | 3,00 |
| Eusolex 6300 | | | (1) | 1,00 |
| (4-Methylbenzyliden Camphor) | | | | |
| IR 3535^{™} (Art.-Nr. 111887) | | | (1) | 10,00 |
| (Ethyl Butylacetylaminopropionate) | | | | |
| (-)-α-Bisabolol (Art.-Nr.130170) | | | (1) | 0,30 |
| Montanov 68 | | | (2) | 4,00 |
| (Cetearyl Alcohol (and) Cetearyl Glucoside) | | | | |
| Myritol 312 | | | (3) | 2,00 |
| (Carprylic/ apric Triglyceride) | | | | |
| Mirasil | CM | 5 | (4) | 2,00 |
| (Cyclomethicone) | | | | |
| Mirasil | DM | 350 | (4) | 1,00 |
| (Dimethicone) | | | | |
| | | | | |

| B | | | | % |
|---|---|---|---|---|
| Demin. Wasser | | | ad | 100,00 |
| Glycerin, 87%ig (Art.-Nr. 104091) | | | (1) | 3,00 |
| Konservierungsmittel | | | | q.s. |
| | | | | |

| C | | | | % |
|---|---|---|---|---|
| Rhodicare | | S | (4) | 0,50 |
| (Xanthan Gum) | | | | |

### Herstellung:

Phasen A und B getrennt auf 75°C erhitzen. C bei 75°C unter Rühren langsam zu B geben und rühren bis eine homogene Mischung entsteht. Anschließend A unter Rühren zu B/C geben und homogenisieren. Unter Rühren abkühlen.

### Bemerkungen:

Als Konservierungsmittel sind enthalten:
0,05% Propyl-4-hydroxybenzoat (Merck KGaA, Art.-Nr. 130173),
0,15% Methyl-4-hydroxybenzoat (Merck KGaA, Art.-Nr. 130174),
0,30% Germall 115 (ISP, Frechen)

### Bezugsquellen:

(1) Merck KGaA, Darmstadt
(2) Interorgana, Köln
(3) Henkel, KGaA, Düsseldorf
(4) Rhodia, Frankfurt

### Beispiel 5

**Herstellung einer Sonnenschutzmilch mit IR3535^{™}**

| A | | % |
|---|---|---|
| 2-[4[(Ethyl) phenyl]-1*H*-imidazo [4,5-*b*]chinoxalin | (1) | 1,00 |
| Eusolex 6300 | (1) | 1,00 |
| (4-Methylbenzyliden Camphor) | | |
| IR 3535^{™} (Art.-Nr. 111887) | (1) | 15,00 |
| (Ethyl Butylacetylaminopropionate) | | |
| Eusolex 2292 | (1) | 3,00 |
| (Octyl Methoxycinnamate) | | |
| Dow Corning 5225 C | (2) | 12,00 |
| (Cyclomethicone (and) Dimethicone Copolyol) | | |
| Dow Corning 345 | (2) | 5,00 |
| (Cyclomethicone) | | |
| Gilugel Sil 5 | (3) | 12,00 |
| (Cyclomethicone (and) AI/Mg Hydroxyde Stearate) | | |
| Solvent ID | (4) | 13,00 |
| (Isododecane) | | |
| Wiconol 14 | (5) | 2,50 |
| (Polyglyeryl-4 Oleate) | | |
| Bienenwachs, gebleicht (Art.-Nr. 111544) | (1) | 1,60 |
| Carnaubawachs | (6) | 0,40 |
| | | |

| B | | % |
|---|---|---|
| Demin. Wasser | ad | 100,00 |
| Propanediol-1,2 (Art.-Nr. 107478) | (1) | 2,00 |
| Natriumchlorid (Art.-Nr. 106400) | (1) | 2,00 |
| Konservierungsmittel | (1) | q.s. |
| | | |

| C | | % |
|---|---|---|
| Parfümöl Bariton (10607) | (7) | 0,30 |

### Herstellung:

Phasen A auf 80°C erhitzen, unter Rühren auf 30°C abkühlen. Phase B unter Rühren langsam zu Phase A geben, rühren bis eine homogene Mischung entsteht und homogenisieren. Phase C zugeben.

### Bemerkungen:

Als Konservierungsmittel ist enthalten:
0,20% Euxyl K400 (Schülke & Mayr, Norderstedt)

### Bezugsquellen:

(1) Merck KGaA, Darmstadt
(2) Dow Corning, Düsseldorf
(3) Nordmann, Rassmann GmbH&Co, Hamburg
(4) BP, Düsseldorf
(5) Witco Chemical, Frankfurt
(6) Aug. Schmidt Nachfolger, Bremen
(7) Haarmann & Reimer, Holzminden

### Beispiel 6 - 9:

Folgende Rezepturen können mit allen erfindungsgemäßen Chinoxalinderivaten hergestellt werden.

| | Substanz | lNCl | 6 | 7 | 8 | 9 |
|---|---|---|---|---|---|---|
| **A** | Chinoxalinderivat | --- | 2,0 | 2,0 | 2,0 | 2,0 |
| | Eusolex^{®} 6300 | 4-METHYLBENZYLIDENE CAMPHOR | 4,0 | - | - | - |
| | Eusolex^{®} 2292 | OCTYL METHOXYCINNAMATE, BHT | - | 6,0 | 7,0 | - |
| | Eusolex^{®} 9020 | BUTYL METHOXYDIBENZOYLMET HANE | - | 1,0 | - | - |
| | Eusolex^{®} OCR | OCTOCRYLENE | - | - | - | 7,0 |
| | Hostaphat CG 120 | ISOSTEARYL PHOSPHATE | 3,00 | 3,00 | 3,00 | 3,00 |
| | Cetiol SN | CETEARYL ISONONAOATE | 5,00 | 4,00 | 4,00 | 4,00 |
| | Cetiol OE | DICAPRYL ETHER | 5,00 | 4,00 | 4,00 | 4,00 |
| | Cetiol 868 | OCTYL STEARATE | 5,00 | 4,00 | 4,00 | 4,00 |
| | Carbopol Ultrez 10 | CARBOMER | 0,15 | 0,15 | 0,15 | 0,15 |
| | Propyl-4-hydroxybenzoate | PROPYLPARABEN | 0,05 | 0,05 | 0,05 | 0,05 |
| **B** | Glycerol (about 87 %) | GLYCERIN | 5,00 | 5,00 | 5,00 | 5,00 |
| | Tris(hydroxymethy l)aminomethane | TROMETHAMINE | 0,15 | 0,15 | 0,15 | 0,15 |
| | RonaCare^{®} Allantoin | ALLANTOIN | 0,30 | 0,30 | 0,30 | 0,30 |
| | Methyl-4-hydroxybenzoate | METHYLPARABEN | 0,15 | 0,15 | 0,15 | 0,15 |
| | Water, demin. | AQUA (WATER) | Ad 100 | Ad 100 | Ad 100 | Ad 100 |

| | | | | | | |
|---|---|---|---|---|---|---|
| INCl = International nomenclature for Cosmetic Ingredients | | | | | | |

Herstellung: Phase A ohne das Carbopol wird zusammengemischt und auf 80°C erhitzt. Unter Rühren wird das Carbopol zu Phase A zugesetzt. Phase B wird zusammengemischt und unter Rühren zu Phase A zugesetzt. Anschließend wird das Gemisch homogenisiert.

Die hier angegebenen Ausführungsbeispiele belegen exemplarisch die Ausführbarkeit der beanspruchten Erfindung, wobei die vorliegende Erfindung im gesamten beanspruchten Bereich ausführbar ist.

## Patentansprüche

1. Verwendung von Verbindungen ausgewählt aus einer der Formeln IV, V, VI oder VII, 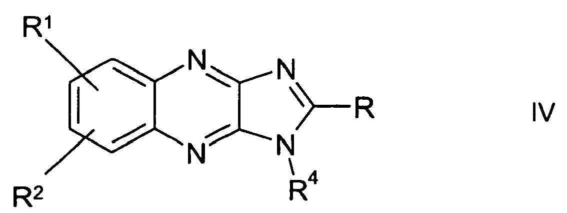 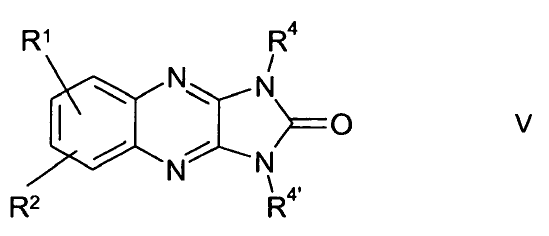 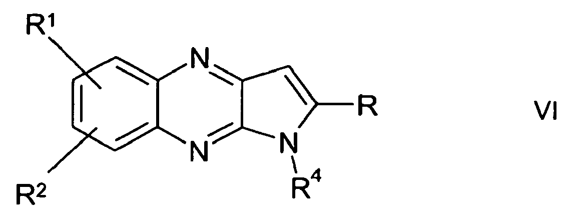 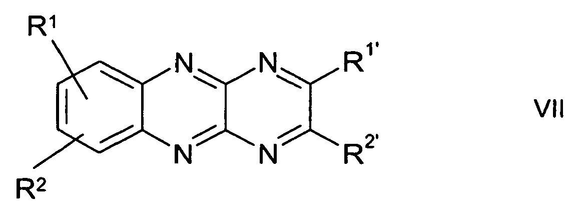 wobei
R, R¹, R², R^{1'} und R^{2'} jeweils unabhängig voneinander H, Alkyl, Alkoxy, Alkenyl oder Alkinyl jeweils mit bis zu 20 C-Atomen, Cycloalkyl, Cycloalkoxy, Cycloalkenyl oder Bicyclische Systeme jeweils mit bis zu 10 C-Atomen, wobei in allen diesen Gruppen auch ein oder mehrere Wasserstoffatome durch Sub¹ substituiert und/oder eine oder zwei CH₂-Gruppen durch C=O ersetzt sein können und in den cyclischen Systemen 1 bis 3 Heteroatome wie S, N und/oder O enthalten sein können, Hal, OH, NO₂, -(CR⁵R⁶)ₙ-NR⁵R⁶, -SO-R⁵, -NR⁵-SO-R⁶, -(CR⁵R⁶)ₙ-N=CR⁵R⁶, -(CR⁵R⁶)ₙ-CR⁵=NR⁵, -SO₂-R⁵, -SR⁵, -(CR⁵R⁶)ₙ-NHCOR⁵, -(CR⁵R⁶)ₙ-NHCOOR⁵,
wasserlöslich machende Substituenten, ausgewählt aus der Gruppe bestehend aus Carboxylat-, Sulfonat- oder Ammoniumresten, COR⁵, COOR⁵, CON⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Si(Alkyl)₃, -Si(Alkyl)₂H, -Het, -NHHet, -OHet oder -(CR⁵R⁶)ₙ-Het,
R¹ und R² bzw. R^{1'} und R^{2'} jeweils zusammen auch mit Kohlenstoffatomen, an die sie gebunden sind, gemeinsam einen ungesättigten, teilweise oder vollständig gesättigten 4-, 5-, 6- oder 7-Ring bilden können, der gegebenenfalls Heteroatome wie S, N und/oder O enthalten kann, weiter anelliert und/oder auch ein- oder mehrfach substituiert sein kann,
Sub¹ Hal, Hydroxy, Cyano, Amino, Nitro, C₁-C₄ -Alkylamino, C₁-C₄-Dialkylamino, C₁-C₄-Alkyl oder C₁-C₄ -Alkoxy, COOH oder COOAlkyl,
Hal Fluor, Chlor, Brom, Jod,
n 0, 1, 2, 3 oder 4,
R⁵, R⁶ jeweils unabhängig voneinander H, Alkyl, Alkenyl, Alkinyl, jeweils mit bis zu 20 C-Atomen, Cycloalkyl, Cycloalkenyl, Bicyclische Systeme, jeweils mit bis zu 10 C-Atomen, wobei diese Reste bis dreifach durch Sub¹ substituiert vorliegen und/oder ein oder zwei CH₂-Gruppen durch C=O ersetzt sein können, und die cyclischen Systeme auch 1 bis 3 Heteroatome wie S, N, und/oder O enthalten können,
-(CR'R")ₙ-Ar oder -(CR'R")ₙ-Het,
die Reste R⁵ und R⁶ auch untereinander, jeweils zusammen mit C-Atomen, an die sie gebunden sind, gemeinsam einen ungesättigten, teilweise oder vollständig gesättigten 4-, 5-, 6-oder 7-Ring bilden können, der gegebenenfalls Heteroatome wie S, N und/oder O enthalten kann, auch ein- oder mehrfach substituiert und/oder weiter anelliert sein kann,
R und R" jeweils unabhängig voneinander H oder C₁-C₄-Alkyl, wobei auch ein oder zwei CH₂-Gruppen durch C=O ersetzt sein können,
Ar ein unsubstituierter oder ein- oder mehrfach substituierter aromatischer Ring oder kondensierte Ringsysteme mit 6 bis 18 C-Atomen, worin auch ein oder zwei CH-Gruppen durch C=O ersetzt sein können,
Het ein unsubstituierter oder ein-oder mehrfach substituierter heteroaromatischer Ring mit 5 bis 7 Ringgliedern oder ein kondensiertes Ringsystem, wobei als Heteroatome ein oder mehrere N-, S- und/oder O-Atome enthalten sind und worin auch ein oder zwei CH-Gruppen in α- oder β-Position zu den Heteroatomen durch C=O ersetzt sein können,
R⁴ und R^{4'} jeweils unabhängig voneinander stehen für H, Alkyl, Alkoxy, Alkenyl, Alkinyl, jeweils mit bis zu 20 C-Atomen, Cycloalkyl, Cycloalkoxy, Cycloalkenyl, Bicyclische Systeme, jeweils mit bis zu 10 C-Atomen, wobei diese Reste bis dreifach durch Sub² substituiert vorliegen und/oder ein oder zwei CH₂-Gruppen durch C=O ersetzt sein können und wobei die cyclischen Systeme auch 1 bis 3 Heteroatome wie S, N, und/oder O enthalten können,
Sub² Hal, Hydroxy, Cyano, Amino, Nitro, C₁-C₄-Alkyl oder C₁-C₄ -Alkoxy, COR⁵, COOR⁵, OAr, OHet, -(CR⁵R⁶)ₙ-Ar oder -(CR⁵R⁶)ₙ-Het, -(CR⁵R⁶)ₙ-NR⁵R⁶, CONR⁵R⁶, CN, O=S(-R⁵)=O O=S(-OR⁵)=O, O=S(-NR⁵R⁶)=O oder R⁵OP(-OR⁶)=O,
bedeuten,
als photostabile UV-Filter.

2. Verwendung von Chinoxalinderivaten nach Anspruch 1, **dadurch gekennzeichnet, dass** R¹, R², R^{1'}, R^{2'}, R^{4'} und R⁴ für H stehen und R vorzugsweise steht für H, Methyl, ^{t}Butyl oder einen Phenylring, der ein oder mehrfach substituiert sein kann mit Gruppen, die eine der in Anspruch 1 für R¹ angegebenen Bedeutungen haben.

3. Verwendung von Chinoxalinderivaten nach mindestens einem der vorstehenden Ansprüche, **dadurch gekennzeichnet, dass** das Chinoxalinderivat ausgewählt ist aus den Verbindungen gemäß Formel VIII, 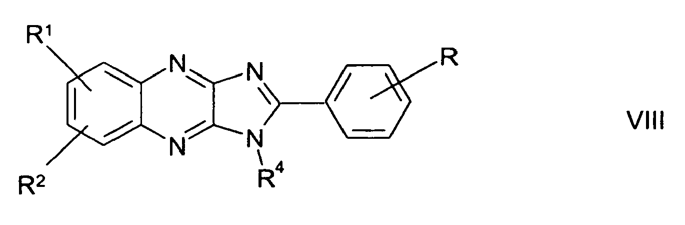 wobei,
R, R1 und R2 die in Anspruch 1 gegebene Bedeutung haben,
R4 steht für H oder einen verzweigten oder unverzweigten C1-20-Alkylrest, in dem gegebenenfalls ein oder mehrere H-Atome durch Sub ersetzt sein können,
Sub steht für H, Methyl, Ethyl oder Ethylhexyl und
R4 vorzugsweise steht für H, Methyl, Ethyl oder Ethylhexyl und
R vorzugsweise steht für einen verzweigten oder unverzweigten C1-20-Alkylrest, in dem gegebenenfalls ein oder mehrere H-Atome durch Sub ersetzt sein können, insbesondere bevorzugt für Methyl, Ethyl, iso-Propyl oder tertiär Butyl.

4. Verbindung gemäß Formel VIII 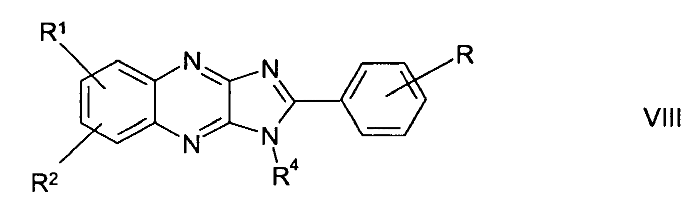 wobei,
R1 und R2 die in Anspruch 1 gegebene Bedeutung haben,
R4 steht für H oder einen verzweigten oder unverzweigten C1-20-Alkylrest, in dem gegebenenfalls ein oder mehrere H-Atome durch Sub ersetzt sein können,
Sub steht für H, Methyl, Ethyl oder Ethylhexyl und
R4 vorzugsweise steht für H, Methyl, Ethyl oder Ethylhexyl und
R im Phenylring steht für
| R | m | Position | 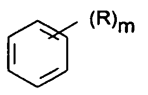 |
|---|---|---|---|
| CH₃ | 2- 3 | o/ m/ p; m/ m/ p; o/p; m/ m | |
| C₂H₅ | 1-3 | " | |
| C₃H₇ | 1-3 | " | |
| ⁿC₄H₉ | 1-3 | " | |
| ^{t}C₄H₉ | 1-3 | " | |
| ⁱC₄H₉ | 1-3 | " | |
| C₅H₁₁ | 1-3 | " | |
| C₁₈H₃₇ | 1-3 | " | |
| (2'-Etyl)-Hexyl- | 1-3 | " | |
| OCH₃ | 3 | o/ m/ p ; m/ m/ p; o/p; m/ m | |
| OC₂H₅ | 1-3 | " | |
| OC₃H₇ | 1-3 | " | |
| OC₄H₉ | 1-3 | " | |
| OC₅H₁₁ | 1-3 | " | |
| OC₁₈H₃₇ | 1-3 | " | |
| OCOCH₃ | 1-2 | o; m; p; o/p | |
| OCOC₂H₅ | 1-2 | " | |
| OCOC₃H₇ | 1-2 | " | |
| OCOC₄H₉ | 1-2 | " | |
| OCOC₅H₁₁ | 1-2 | " | |
| OCOC₁₈H₃₇ | 1-2 | " | |
| OH | 2-3 | o/ m/ p; m/ m/ p; m/ m | |
| F | 1-2 | o; p; o/p | |
| Cl | 1-2 | o; p; o/p | |
| CF₃ | 1 | o; m; p | |
| NO₂ | 2-3 | m/ m; o/ o/ p | |
| NHCOR⁵ | 1 | P | |
| NHCO0R⁵ | 1 | P | |
| COR⁵ | 1-2 | o; p; o/p | |
| COOR⁵ | 1-2 | o; p; o/p | |
| CONR⁵R⁶ | 1-2 | o; p; o/p | |
| CN | 1 | P | |
| O=S(OR⁵)=O | 1 | P | |
| O=S(R⁵)=O | 1 | P | |
| O=S(NR⁵R⁶)=O | 1 | P | |
| R⁵OP(-OR⁶)=O | 1 | P | |

5. Verbindung, **dadurch gekennzeichnet, dass** die Verbindung ausgewählt ist aus den Verbindungen

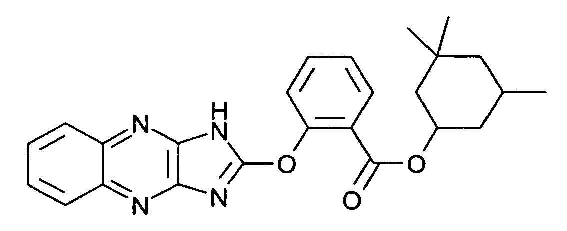

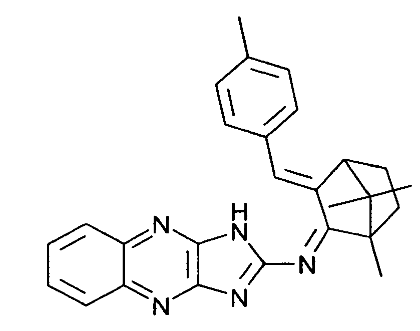

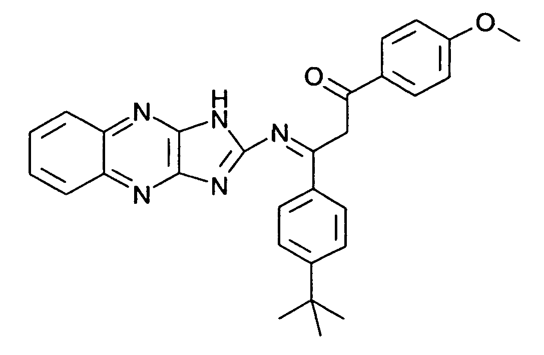

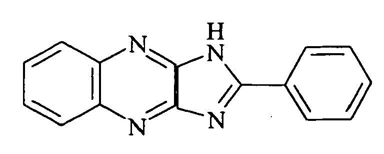

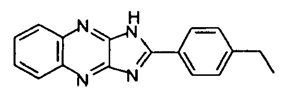

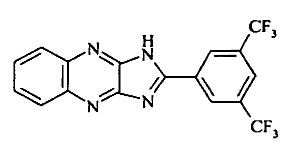

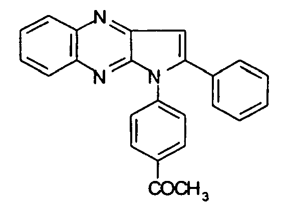

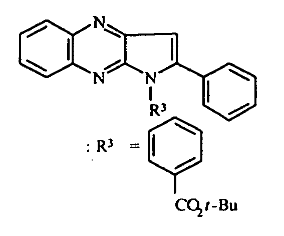

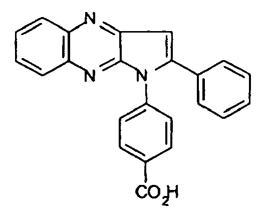

6. Verfahren zur Herstellung von Verbindungen gemäß Anspruch 4 oder 5, **dadurch gekennzeichnet, dass** von einem 2,3-Dichlorchinoxalin, das entsprechend der Zielverbindung substituiert ist, ausgegangen wird.

7. Verwendung von Verbindungen ausgewählt ist aus einer der Formeln IV, V, VI oder VII gemäß Anspruch 1 als UV-Stabilisatoren in kosmetischen und pharmazeutischen Zubereitungen.

8. Verwendung von Verbindungen ausgewählt ist aus einer der Formeln IV, V, VI oder VII gemäß Anspruch 1 zur Photostabilisierung von UV-Filtern, insbesondere Dibenzoylmethanderivaten, wie vorzugsweise 4-t-Butyl-4'-methoxy-dibenzoylmethan.

9. Lichtschutzmittel enthaltende kosmetische und pharmazeutische Zubereitungen zum Schutz der menschlichen Epidermis oder menschlichen Haare gegen UV-Licht im Bereich von 280 bis 400 nm, **dadurch gekennzeichnet, dass** sie in einem kosmetisch und pharmazeutisch geeigneten Träger, zusammen mit an sich für kosmetische und pharmazeutische Zubereitungen bekannten im UV-Bereich absorbierenden Verbindungen, als photostabile UV-Filter wirksame Mengen mindestens einer Verbindung ausgewählt aus einer der Formeln IV, V, VI oder VII, 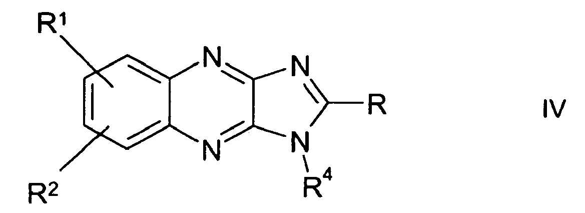 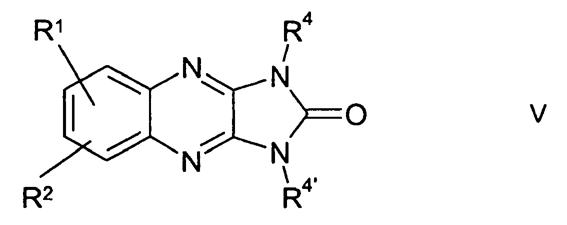 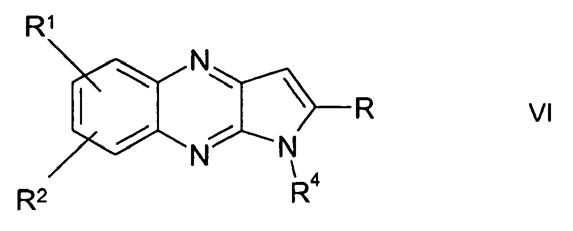 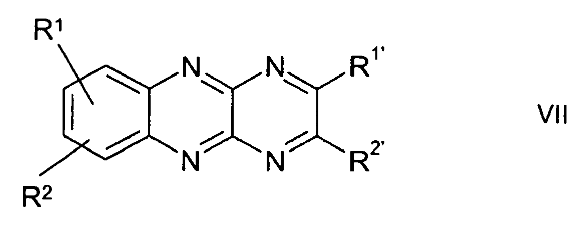 enthalten, in der die Variablen die Bedeutung gemäß Anspruch 1 haben.

10. Lichtschutzmittel enthaltende kosmetische und pharmazeutische Zubereitungen gemäß Anspruch 9, **dadurch gekennzeichnet dass** als UV-Filter Verbindungen der Formel VIII mit Bedeutung der Reste gemäß Anspruch 3 enthalten sind.

11. Kosmetische oder pharmazeutische Zubereitung nach mindestens einem der Ansprüche 9 oder 10, wobei die Zubereitung einen oder mehrere UV-Filter enthält, die ausgewählt sind aus der Gruppe, die 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze enthält.

12. Kosmetische oder pharmazeutische Zubereitung nach mindestens einem der Ansprüche 9 bis 11, **dadurch gekennzeichnet, dass** ein oder mehrere Antixidantien und/oder mindestens eine der Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) und/oder mindestens ein Aryloxim, vorzugsweise 2-Hydroxy5-methyllaurophenonoxim und/oder ein Coumaranonderivat, vorzugsweise 4,6,3',4'-Tetrahydroxybenzylcoumaranon-3 oder dessen Trisulfat enthalten sind.

## Claims

1. Use of compounds selected from one of the formulae IV, V, VI or VII 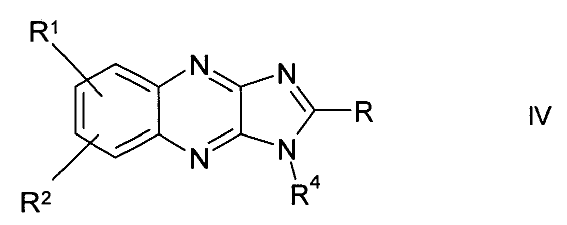 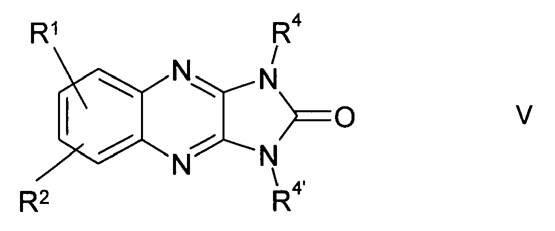 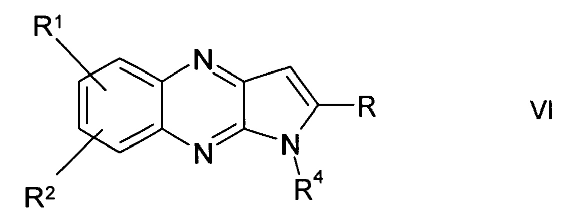 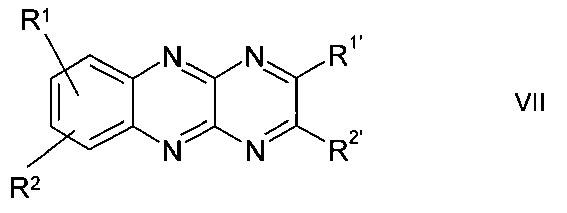 where
R, R¹, R², R^{1'} and R^{2'} each, independently of one another, denote H, alkyl, alkoxy, alkenyl or alkynyl, each having up to 20 C atoms, cycloalkyl, cycloalkoxy, cycloalkenyl or bicyclic systems, each having up to 10 C atoms, where, in all of these groups, one or more hydrogen atoms may also be substituted by Sub¹ and/or one or two CH₂ groups may be replaced by C=O, and 1 to 3 heteroatoms, such as S, N and/or O, may be present in the cyclic systems, Hal, OH, NO₂, -(CR⁵R⁶)ₙ-NR⁵R⁶, -SO₂-R⁵, -NR⁵-SO-R⁶, -(CR⁵R⁶)ₙ-N=CR⁵R⁶, -(CR⁵R⁶)ₙ-CR⁵=NR⁵, -SO-R⁵, -SR⁵, -(CR⁵R⁶)ₙ-NHCOR⁵, -(CR⁵R⁶)ₙ-NHCOOR⁵,
water-solubilising substituents selected from the group consisting of carboxylate, sulfonate or ammonium radicals, COR⁵, COOR⁵, CON⁵R⁶, CN, O=S(-R⁵)=O, O=S(-OR⁵)=O, O=S(-NR⁵R⁶)=O, R⁵OP(-OR⁶)=O, OAr, -(CR⁵R⁶)ₙ-Ar, -Si(alkyl)₃, -Si(alkyl)₂H, -Het, -NHHet, -OHet or -(CR⁵R⁶)ₙ-Het,
R¹ and R² or R^{1'} and R^{2'} in each case together, also with carbon atoms to which they are bonded, may jointly form an unsaturated, partially or fully saturated 4-, 5-, 6- or 7-membered ring, which may optionally contain heteroatoms, such as S, N and/or O, may be further fused and/or may also be mono- or polysubstituted,
Sub¹ denotes Hal, hydroxyl, cyano, amino, nitro, C₁-C₄-alkylamino, C₁-C₄-dialkylamino, C₁-C₄-alkyl or C₁-C₄-alkoxy, COOH or COO-alkyl,
Hal denotes fluorine, chlorine, bromine, iodine,
n denotes 0, 1, 2, 3 or 4,
R⁵, R⁶ each, independently of one another, denote H, alkyl, alkenyl, alkynyl, each having up to 20 C atoms, cycloalkyl, cycloalkenyl, bicyclic systems, each having up to 10 C atoms, where these radicals may be up to trisubstituted by Sub¹ and/or one or two CH₂ groups may be replaced by C=O, and the cyclic systems may also contain 1 to 3 heteroatoms, such as S, N and/or O,
-(CR'R")ₙ-Ar or -(CR'R")ₙ-Het,
the radicals R⁵ and R⁶ may also, with one another, in each case together with C atoms to which they are bonded, jointly form an unsaturated, partially or fully saturated 4-, 5-, 6- or 7-membered ring, which may optionally contain heteroatoms, such as S, N and/or O, may also be mono- or polysubstituted and/or may be further fused,
R' and R" each, independently of one another, denote H or C₁-C₄-alkyl, where one or two CH₂ groups may also be replaced by C=O,
Ar denotes an unsubstituted or mono- or polysubstituted aromatic ring or condensed ring systems having 6 to 18 C atoms, in which one or two CH groups may also be replaced by C=O,
Het denotes an unsubstituted or mono- or polysubstituted heteroaromatic ring having 5 to 7 ring members or a condensed ring system, where one or more N, S and/or O atoms may be present as heteroatoms and in which one or two CH groups in the α- or β-position to the heteroatoms may also be replaced by C=O,
R⁴ and R⁴' each, independently of one another, stand for H, alkyl, alkoxy, alkenyl, alkynyl, each having up to 20 C atoms, cycloalkyl, cycloalkoxy, cycloalkenyl, bicyclic systems, each having up to 10 C atoms, where these radicals may be up to trisubstituted by Sub² and/or one or two CH₂ groups may be replaced by C=O and where the cyclic systems may also contain 1 to 3 heteroatoms, such as S, N and/or O,
Sub² denotes Hal, hydroxyl, cyano, amino, nitro, C₁-C₄-alkyl or C₁-C₄-alkoxy, COR⁵, COOR⁵, OAr, OHet, -(CR⁵R⁶)ₙ-Ar or -(CR⁵R⁶)ₙ-Het, -(CR⁵R⁶)ₙ-NR⁵R⁶, CONR⁵R⁶, CN, O=S-(-R⁵)=O, O=S(-OR⁵)=O, O=S(-NR⁵R⁶)=O or R⁵OP-(-OR⁶)=O,
as photostable UV filters.

2. Use of quinoxaline derivatives according to Claim 1, **characterised in that** R¹, R², R^{1'}, R^{2'}, R^{4'} and R⁴ stand for H, and R preferably stands for H, methyl, ^{t}butyl or a phenyl ring, which may be mono- or polysubstituted by groups which have one of the meanings indicated for R¹ in Claim 1.

3. Use of quinoxaline derivatives according to at least one of the preceding claims, **characterised in that** the quinoxaline derivative is selected from the compounds of the formula VIII 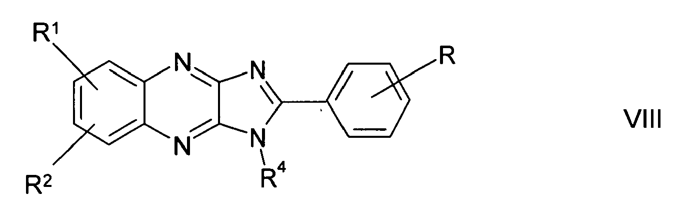 where
R, R¹ and R² have the meaning given in Claim 1,
R⁴ stands for H or a branched or unbranched C₁₋₂₀-alkyl radical, in which one or more H atoms may optionally be replaced by Sub,
Sub stands for H, methyl, ethyl or ethylhexyl and
R⁴ preferably stands for H, methyl, ethyl or ethylhexyl and
R preferably stands for a branched or unbranched C₁₋₂₀-alkyl radical, in which one or more H atoms may optionally be replaced by Sub, particularly preferably for methyl, ethyl, isopropyl or tertiary butyl.

4. Compound of the formula VIII 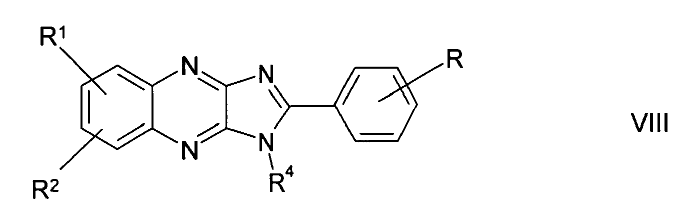 where
R¹ and R² have the meaning given in Claim 1,
R⁴ stands for H or a branched or unbranched C₁₋₂₀-alkyl radical, in which one or more H atoms may optionally be replaced by Sub,
Sub stands for H, methyl, ethyl or ethylhexyl and
R⁴ preferably stands for H, methyl, ethyl or ethylhexyl and
R in the phenyl ring stands for
| R | m | Position | 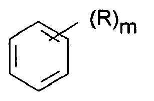 |
|---|---|---|---|
| CH₃ | 2-3 | o/ m/ p; m/ m/ p; o/p; m/ m | |
| C₂H₅ | 1-3 | " | |
| C₃H₇ | 1-3 | " | |
| ⁿC₄H₉ | 1-3 | " | |
| ^{t}C₄H₉ | 1-3 | " | |
| ⁱC₄H₉ | 1-3 | " | |
| C₅H₁₁ | 1-3 | " | |
| C₁₈H₃₇ | 1-3 | " | |
| (2'-Ethyl)-hexyl- | 1-3 | " | |
| OCH₃ | 3 | o/ m/ p; m/ m/ p; o/p; m/ m | |
| OC₂H₅ | 1-3 | " | |
| OC₃H₇ | 1-3 | " | |
| OC₄H₉ | 1-3 | " | |
| OC₅H₁₁ | 1-3 | " | |
| OC₁₈H₃₇ | 1-3 | " | |
| OCOCH₃ | 1-2 | o; m; p; o/p | |
| OCOC₂H₅ | 1-2 | " | |
| OCOC₃H₇ | 1-2 | " | |
| OCOC₄H₉ | 1-2 | " | |
| OCOC₅H₁₁ | 1-2 | " | |
| OCOC₁₈H₃₇ | 1-2 | " | |
| OH | 2-3 | o/ m/ p; m/ m/ p; m/ m | |
| F | 1-2 | o; p; o/p | |
| Cl | 1-2 | o; p; o/p | |
| CF₃ | 1 | o; m; p | |
| NO₂ | 2-3 | m/ m; o/ o/ p | |
| NHCOR⁵ | 1 | p | |
| NHCOOR⁵ | 1 | p | |
| COR⁵ | 1-2 | o; p; o/p | |
| COOR⁵ | 1-2 | o; p; o/p | |
| CONR⁵R⁶ | 1-2 | o; p; o/p | |
| CN | 1 | p | |
| O=S(OR⁵)=O | 1 | p | |
| O=S(R⁵)=O | 1 | p | |
| O=S(NR⁵R⁶)=O | 1 | p | |
| R⁵OP(-OR⁶)=O | 1 | p | |

5. Compound, **characterised in that** the compound is selected from the compounds

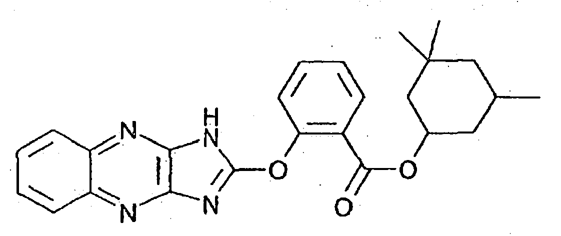

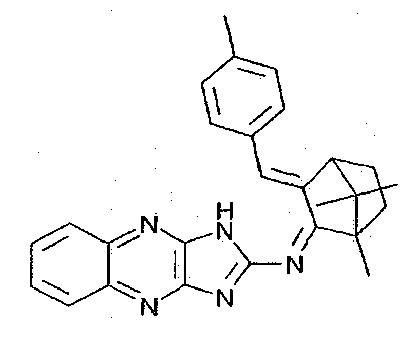

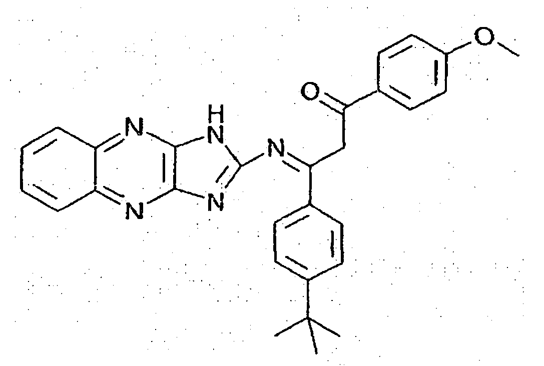

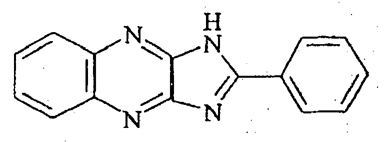

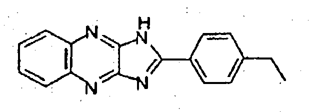

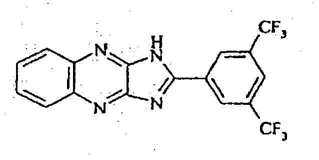

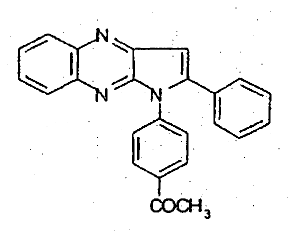

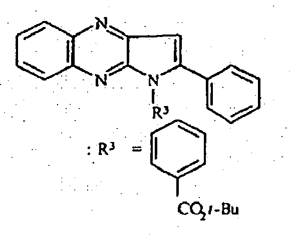

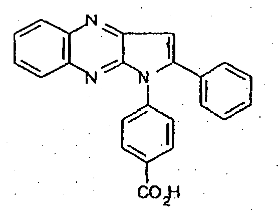

6. Process for the preparation of compounds according to Claim 4 or 5, **characterised in that** the starting material is a 2,3-dichloroquinoxaline which is substituted corresponding to the target compound.

7. Use of compounds selected from one of the formulae IV, V, VI or VII according to Claim 1 as UV stabilisers in cosmetic and pharmaceutical compositions.

8. Use of compounds selected from one of the formulae IV, V, VI or VII according to Claim 1 for the photostabilisation of UV filters, in particular dibenzoylmethane derivatives, such as preferably 4-t-butyl-4'-methoxydibenzoylmethane.

9. Cosmetic and pharmaceutical compositions comprising light-protection agents for protection of the human epidermis or human hair against UV light in the range from 280 to 400 nm, **characterised in that** they comprise, as photostable UV filters, effective amounts of at least one compound selected from one of the formulae IV, V, VI or VII 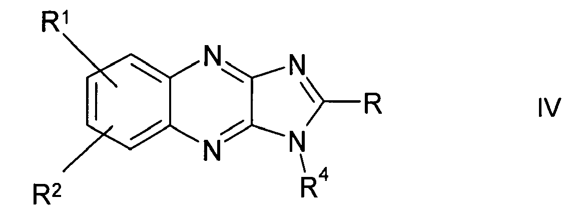 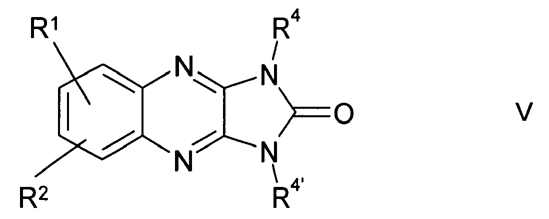 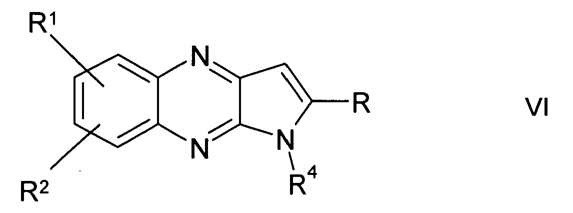 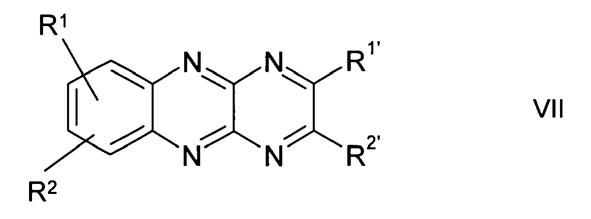 in which the variables have the meaning according to Claim 1, in a cosmetically and pharmaceutically suitable vehicle, together with compounds absorbent in the UV region which are known per se for cosmetic and pharmaceutical compositions.

10. Cosmetic and pharmaceutical compositions according to Claim 9 comprising light-protection agents, **characterised in that** compounds of the formula VIII, where the radicals have the meaning given in Claim 3, are present as UV filters.

11. Cosmetic or pharmaceutical composition according to at least one of Claims 9 or 10, where the composition comprises one or more UV filters selected from the group comprising 3-(4'-methylbenzylidene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and potassium, sodium and triethanolamine salts thereof.

12. Cosmetic or pharmaceutical composition according to at least one of Claims 9 to 11, **characterised in that** one or more antioxidants and/or at least one of the pyrimidinecarboxylic acids ectoin ((S)-1,4,5,6-tetrahydro-2-methyl-4-pyrimidinecarboxylic acid) and hydroxyectoin ((S, S)-1,4,5,6-tetrahydro-5-hydroxy-2-methyl-4-pyrimidinecarboxylic acid) and/or at least one aryl oxime, preferably 2-hydroxy-5-methyllaurophenone oxime, and/or a coumaranone derivative, preferably 4,6,3',4'-tetrahydroxybenzyl-3-coumaranone or its trisulfate, are present.

## Revendications

1. Utilisation de composés choisis parmi l'une des formules IV, V, VI ou VII 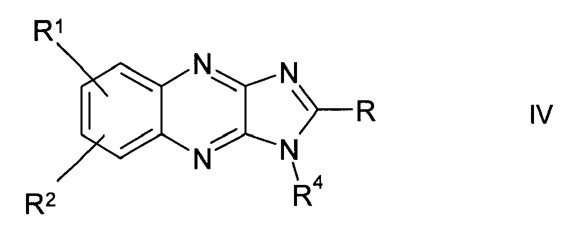 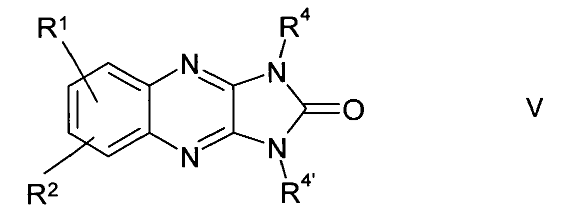 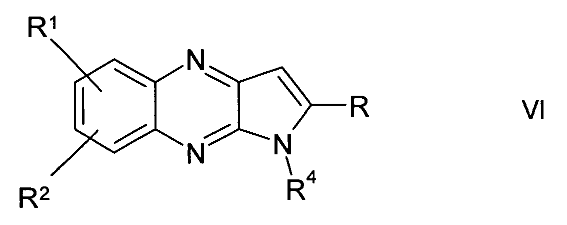 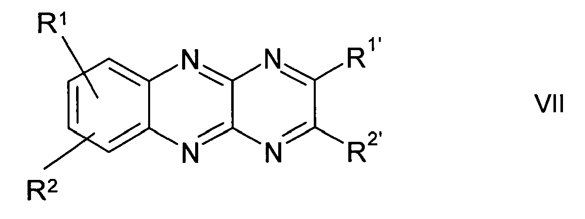 dans lesquelles
R, R¹, R², R^{1'} et R^{2'} désignent chacun, indépendamment l'un de l'autre, H, alkyle, alcoxy, alcényle ou alcynyle, ayant chacun jusqu'à 20 atomes de C, cycloalkyle, cycloalcoxy, cycloalcényle ou des systèmes bicycliques, ayant chacun jusqu'à 10 atomes de C, où, dans tous ces groupements, un ou plusieurs atomes d'hydrogène peuvent également être substitués par Sub¹ et/ou un ou deux groupements CH₂ peuvent être remplacés par C=O, et 1 à 3 hétéroatomes, tels que S, N et/ou O, peuvent être présents dans les systèmes cycliques, Hal, OH, NO₂, -(CR⁵R⁶)ₙ-NR⁵R⁶, -SO₂-R⁵, -NR⁵-SO-R⁶, -(CR⁵R⁶)ₙ-N=CR⁵R⁶, -(CR⁵R⁶)ₙ-CR⁵=NR⁵, -SO-R⁵, -SR⁵, -(CR⁵R⁶)ₙ-NHCOR⁵, -(CR⁵R⁶)ₙ-NHCOOR⁵,
des substituants hydrosolubilisants choisis parmi le groupe constitué par des radicaux carboxylate, sulfonate ou ammonium, COR⁵, COOR⁵, CON⁵R⁶, CN, O=S-(-R⁵)=O, O=S(-OR⁵)=O, O=S(-NR⁵R⁶)=O, R⁵OP(-OR⁶)=O, OAr, -(CR⁵R⁶)ₙ-Ar, -Si(alkyl)₃, -Si(alkyl)₂H, -Hét, -NHHét, -OHét ou -(CR⁵R⁶)ₙ-Hét,
R¹ et R² ou R^{1'} et R^{2'} dans chaque cas, ensemble, également avec les atomes de carbone auxquels ils sont liés, peuvent former ensemble un cycle insaturé, partiellement ou totalement saturé de 4, 5, 6 ou 7 chaînons, pouvant éventuellement contenir des hétéroatomes, tels que S, N et/ou O, pouvant être en outre condensé et/ou pouvant également être mono- ou polysubstitué,
Sub¹ désigne Hal, hydroxyle, cyano, amino, nitro, alkyl(C₁-C₄)-amino, dialkyl(C₁-C₄)amino, alkyl(C₁-C₄) ou alcoxy(C₁-C₄), COOH ou COO-alkyle,
Hal désigne fluor, chlore, brome, iode,
n vaut 0, 1, 2, 3 ou 4,
R⁵, R⁶ désignent chacun, indépendamment l'un de l'autre, H, alkyle, alcényle, alcynyle, ayant chacun jusqu'à 20 atomes de C, cycloalkyle, cycloalcényle, des systèmes bicycliques, ayant chacun jusqu'à 10 atomes de C, où ces radicaux peuvent être jusqu'à trisubstitués par Sub¹ et/ou un ou deux groupements CH₂ peuvent être remplacés par C=O, et les systèmes cycliques peuvent également contenir 1 à 3 hétéroatomes, tels que S, N et/ou O,
-(CR'R")ₙ-Ar ou -(CR'R")ₙ-Hét,
les radicaux R⁵ et R⁶ peuvent également, l'un avec l'autre, dans chaque cas conjointement avec les atomes de C auxquels ils sont fixés, former ensemble un cycle insaturé, partiellement ou totalement saturé de 4, 5, 6 ou 7 chaînons, pouvant éventuellement contenir des hétéroatomes, tels que S, N et/ou O, pouvant également être mono- ou polysubstitué et/ou pouvant être en outre condensé,
R' et R" désignent chacun, indépendamment l'un de l'autre, H ou alkyl(C₁-C₄), où un ou deux groupements CH₂ peuvent également être remplacés par C=O,
Ar désigne un cycle aromatique non substitué ou mono- ou polysubstitué ou des noyaux condensés ayant 6 à 18 atomes de C, dans lesquels un ou deux groupements CH peuvent également être remplacés par C=O,
Hét désigne un cycle hétéroaromatique non substitué ou mono- ou polysubstitué ayant 5 à 7 chaînons de cycle ou un noyau condensé, où un ou plusieurs atomes de N, S et/ou O peuvent être présents sous forme d'hétéroatomes et dans lequel un ou deux groupements CH en position α ou β par rapport aux hétéroatomes peuvent également être remplacés par C=O,
R⁴ et R⁴' représentent chacun, indépendamment l'un de l'autre, H, alkyle, alcoxy, alcényle, alcynyle, ayant chacun jusqu'à 20 atomes de C, cycloalkyle, cycloalcoxy, cycloalcényle, des systèmes bicycliques, ayant chacun jusqu'à 10 atomes de C, où ces radicaux peuvent être jusqu'à trisubstitués par Sub² et/ou un ou deux groupements CH₂ peuvent être remplacés par C=O et où les systèmes cycliques peuvent également contenir 1 à 3 hétéroatomes, tels que S, N et/ou O,
Sub² désigne Hal, hydroxyle, cyano, amino, nitro, alkyl(C₁-C₄) ou alcoxy(C₁-C₄), COR⁵, COOR⁵, OAr, OHét, -(CR⁵R⁶)ₙ-Ar ou -(CR⁵R⁶)ₙ-Hét, -(CR⁵R⁶)ₙ-NR⁵R⁶, CONR⁵R⁶, CN, O=S(-R⁵)=O, O=S(-OR⁵)=O, O=S(-NR⁵R⁶)=O ou R⁵OP-(-OR⁶)=O,
comme filtres UV photostables.

2. Utilisation de dérivés de quinoxaline selon la revendication 1, **caractérisée en ce que** R¹, R², R¹', R²' , R⁴' et R⁴ représentent H, et R représente préférablement H, méthyle, ^{t}butyle ou un cycle phényle, pouvant être mono- ou polysubstitué par des groupements ayant l'une des significations indiquées pour R¹ selon la revendication 1.

3. Utilisation de dérivés de quinoxaline selon au moins l'une parmi les revendications précédentes, **caractérisée en ce que** le dérivé de quinoxaline est choisi parmi les composés de formule VIII 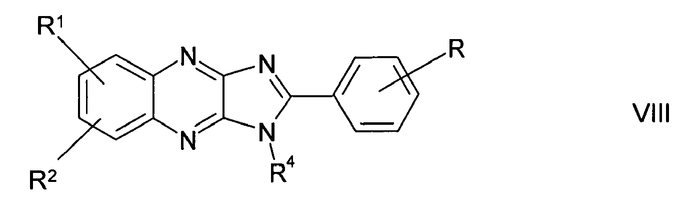 dans laquelle
R, R¹ et R² ont la signification indiquée selon la revendication 1,
R⁴ représente H ou un radical alkyl(C₁₋₂₀) ramifié ou non ramifié, dans lequel un ou plusieurs atomes de H peuvent éventuellement être remplacés par Sub,
Sub représente H, méthyle, éthyle ou éthylhexyle et
R⁴ représente préférablement H, méthyle, éthyle ou éthylhexyle et
R représente préférablement un radical alkyl(C₁₋₂₀) ramifié ou non ramifié, dans lequel un ou plusieurs atomes de H peuvent éventuellement être remplacés par Sub, particulièrement préférablement par méthyle, éthyle, isopropyle ou tertio-butyle.

4. Composé de formule VIII 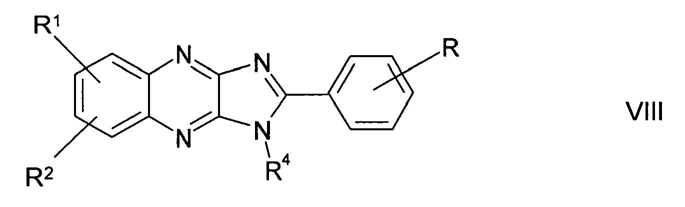 dans laquelle
R¹ et R² ont la signification indiquée selon la revendication 1,
R⁴ représente H ou un radical alkyl(C₁₋₂₀) ramifié ou non ramifié, dans lequel un ou plusieurs atomes de H peuvent éventuellement être remplacés par Sub,
Sub représente H, méthyle, éthyle ou éthylhexyle et
R⁴ représente préférablement H, méthyle, éthyle ou éthylhexyle et
R dans le cycle phényle représente
| R | m | Position | 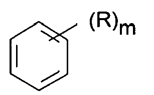 |
|---|---|---|---|
| CH₃ | 2-3 | o/ m/ p ; m/ m/ p ; o/p ; m/ m | |
| C₂H₅ | 1-3 | " | |
| C₃H₇ | 1-3 | " | |
| ⁿC₄H₉ | 1-3 | " | |
| ^{t}C₄H₉ | 1-3 | " | |
| ⁱC₄H₉ | 1-3 | " | |
| C₅H₁₁ | 1-3 | " | |
| C₁₈H₃₇ | 1-3 | " | |
| (2'-Éthyl)-hexyl- | 1-3 | " | |
| OCH₃ | 3 | o/ m/ p ; m/ m/ p ; o/p ; m/ m | |
| OC₂H₅ | 1-3 | " | |
| OC₃H₇ | 1-3 | " | |
| OC₄H₉ | 1-3 | " | |
| OC₅H₁₁ | 1-3 | " | |
| OC₁₈H₃₇ | 1-3 | " | |
| OCOCH₃ | 1-2 | o ; m ; p ; o/p | |
| OCOC₂H₅ | 1-2 | " | |
| OCOC₃H₇ | 1-2 | " | |
| OCOC₄H₉ | 1-2 | " | |
| OCOC₅H₁₁ | 1-2 | " | |
| OCOC₁₈H₃₇ | 1-2 | " | |
| OH | 2-3 | o/ m/ p ; m/ m/ p ; m/ m | |
| F | 1-2 | o ; p ; o/p | |
| Cl | 1-2 | o ; p ; o/p | |
| CF₃ | 1 | o ; m ; p | |
| NO₂ | 2-3 | m/ m ; o/ o/ p | |
| NHCOR⁵ | 1 | p | |
| NHCOOR⁵ | 1 | p | |
| COR⁵ | 1-2 | o ; p ; o/p | |
| COOR⁵ | 1-2 | o ; p ; o/p | |
| CONR⁵R⁶ | 1-2 | o ; p ; o/p | |
| CN | 1 | p | |
| O=S(OR⁵)=O | 1 | p | |
| O=S(R⁵)=O | 1 | p | |
| O=S(NR⁵R⁶)=O | 1 | p | |
| R⁵OP(-OR⁶)=O | 1 | p | |

5. Composé, **caractérisé en ce que** le composé est choisi parmi les composés

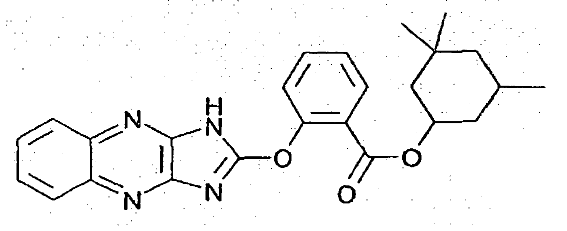

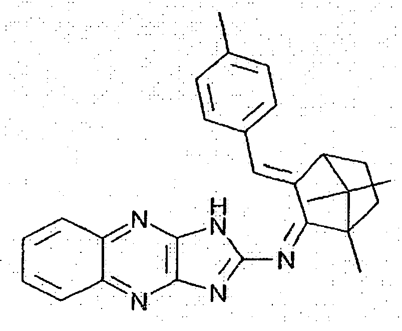

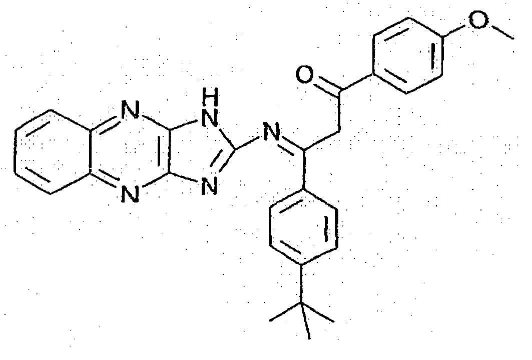

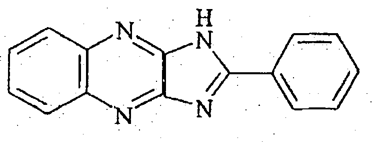

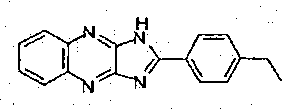

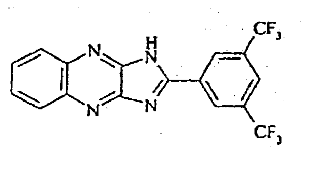

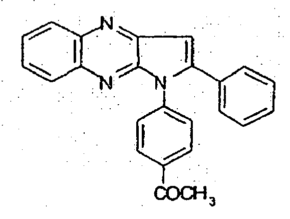

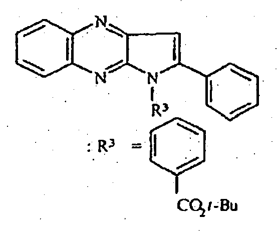

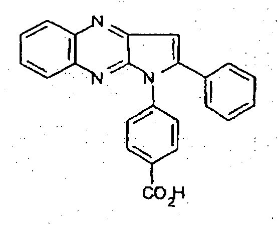

6. Procédé de préparation de composés selon la revendication 4 ou 5, **caractérisé en ce que** le produit de départ est une 2,3-dichloroquinoxaline qui est substituée selon le composé cible.

7. Utilisation de composés choisis parmi l'une des formules IV, V, VI ou VII selon la revendication 1, comme absorbeurs UV dans des compositions pharmaceutiques et cosmétiques.

8. Utilisation de composés choisis parmi l'une des formules IV, V, VI ou VII selon la revendication 1, pour la photostabilisation de filtres UV, en particulier des dérivés de dibenzoylméthane, tels que préférablement le 4-t-butyl-4'-méthoxydibenzoylméthane.

9. Compositions cosmétiques et pharmaceutiques comprenant des agents de protection contre la lumière, destinées à la protection de l'épiderme humain ou des cheveux humains contre la lumière UV dans le domaine allant de 280 à 400 nm, **caractérisées en ce qu'**elles comprennent, comme filtres UV photostables, des quantités efficaces d'au moins un composé choisi parmi l'un des formules IV, V, VI ou VII 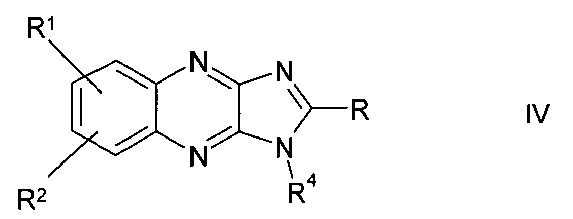 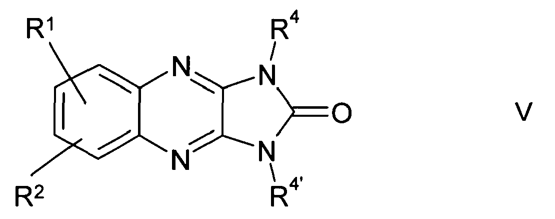 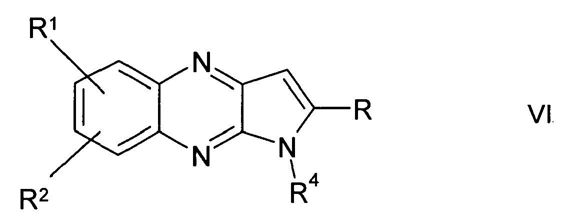 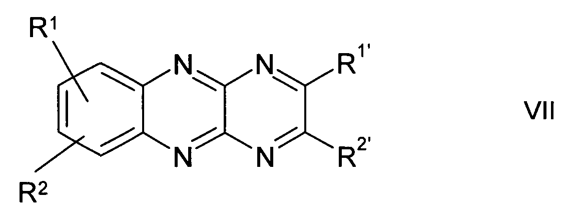 dans lesquelles les variables ont la signification selon la revendication 1, dans un véhicule cosmétiquement et pharmaceutiquement convenable, conjointement avec des composés absorbant dans le domaine UV qui sont connus en soi pour des compositions cosmétiques et pharmaceutiques.

10. Compositions cosmétiques et pharmaceutiques selon la revendication 9 comprenant des agents de protection contre la lumière, **caractérisées en ce que** les composés de formule VIII, où les radicaux ont la signification indiquée selon la revendication 3, sont présents comme filtres UV.

11. Composition cosmétique ou pharmaceutique selon au moins l'une des revendications 9 ou 10, où la composition comprend un ou plusieurs filtres UV sélectionnés parmi le groupe constitué par le 3-(4'-méthylbenzylidène)-dl-camphre, la 1-(4-tertio-butylphényl)-3-(4-méthoxyphényl)propane-1,3-dione, le 4-isopropyldibenzoylméthane, la 2-hydroxy-4-méthoxybenzophénone, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, de sodium et de triéthanolamine de ceux-ci.

12. Composition pharmaceutique ou cosmétique selon au moins l'une des revendications 9 à 11, **caractérisée en ce qu'**un ou plusieurs antioxydants et/ou au moins l'un parmi les acides pyrimidinecarboxyliques, l'ectoïne (acide (S)-1,4,5,6-tétrahydro-2-méthyl-4-pyrimidinecarboxylique) et l'hydroxyectoïne (acide (S, S)-1,4,5,6-tétrahydro-5-hydroxy-2-méthyl-4-pyrimidinecarboxylique), et/ou au moins un aryl oxime, préférablement le 2-hydroxy-5-méthyllaurophénone oxime, et/ou un dérivé de coumaranone, préférablement la 4,6,3',4'-tétrahydroxybenzyl-3-coumaranone ou son trisulfate, sont présents.
